# FASCICULE DE BREVET EUROPEEN

(11) **EP 1 155 140 B1**
(45) Date de publication et mention de la délivrance du brevet: **28.05.2003**
(21) Numéro de dépôt: 00906441.1
(22) Date de dépôt: 21.02.2000
(51) Int. Cl.: C12P 19/34, C12Q 1/68, C07H 21/00

(54) **PROCEDE DE FABRICATION DE MORPHOLINO-NUCLEOTIDES, ET LEUR UTILISATION POUR L'ANALYSE ET LE MARQUAGE DE SEQUENCES D'ACIDES NUCLEIQUES**
VERFAHREN ZUR HERSTELLUNG VON MORPHOLINONUKLEOTIDEN UND VERWENDUNG DERSELBEN ZUR ANALYSE UND MARKIERUNG VON NUKLEINSÄURESEQUENZEN
METHODS FOR MAKING MORPHOLINO-NUCLEOTIDES, AND THEIR USE FOR ANALYSING AND MARKING NUCLEIC ACID SEQUENCES

(30) Priorité: 22.02.1999 FR 9902170; 27.09.1999 FR 9912001
(43) Date de publication de la demande: 21.11.2001
(73) Titulaire: COMMISSARIAT A L'ENERGIE ATOMIQUE, 75752 Paris Cédex 15 (FR); CENTRE NATIONAL DE LA RECHERCHE SCIENTIFIQUE, 75016 Paris Cédex (FR)
(72) Inventeur: MARCIACQ, Florence, F-13580 La Fare Les Oliviers (FR); SAUVAIGO, Sylvie, F-38320 Herbeys (FR); MOURET, Jean-François, F-38500 Coublevie (FR); ISSARTEL, Jean-Paul, F-38120 Saint-Egreve (FR); MOLKO, Didier, F-38210 Tullins (FR)
(74) Mandataire: Audier, Philippe André
(86) Numéro de dépôt international: FR0000427
(87) Numéro de publication internationale: WO00050626

(56) Documents cités:
- WO-A-95/07907
- US-A- 4 515 781
- BROWN ET AL.: "The Reduction of the Adduct of Periodate-oxidised Adenosine-5' Phosphate and Methylamine." J. CHEM. SOC., 1965, pages 5072-5073, XP002121978
- GIRAULT ET AL.: "Use of Morpholinonucleosides to Conjugate Oxidised DNA Bases to Proteins." BIOCONJUGATE CHEM., vol. 7, 1996, pages 445-450, XP002121979
- MARCIACQ ET AL.: "Synthesis and enzymatic incorporation of Morpholino thymidine-5'-triphosphate in DNA fragments." TETRAHEDRON LETT., vol. 40, 18 juin 1999 (1999-06-18), pages 4673-4676, XP004167188

## Description

### Domaine technique

La présente invention a pour objet la fabrication de fragments d'acides nucléiques (ADN ou ARN) allongés enzymatiquement au moyen de morpholino-nucléosides triphosphates. Cette élongation peut être utilisée pour l'analyse de séquences d'acides nucléiques par incorporation de ces dérivés dans des chaînes d'acides nucléiques ainsi que le marquage enzymatique et l'immobilisation ou la détection de séquences.

Ces morpholino-nucléosides triphosphates peuvent encore être utilisés avec une molécule supplémentaire pouvant jouer divers rôles dans de nombreuses applications.

### État de la technique antérieure

La méthode la plus répandue pour analyser les séquences des acides nucléiques est la technique enzymatique dite de terminaison de chaîne, développée par Sanger et al dans Proceedings of National Academy of Science, 74, 1977, p. 5463-5467 [1]. Elle repose sur les propriétés qu'ont les ADN polymérases-ADN dépendantes de créer des polymères d'ADN complémentaires de la séquence d'un brin d'ADN servant de matrice, à partir d'un mélange de monomères de nucléosides triphosphates naturels. Le procédé consiste, à partir du brin d'ADN à analyser, à faire une série d'exemplaires du brin complémentaire en ajoutant au milieu réactionnel classique des molécules appelées « terminateurs de chaînes », puis à analyser la longueur des brins néoformés pour déterminer la séquence des bases de la matrice. Le principe de la méthode est expliqué dans le tableau 1 qui suit.

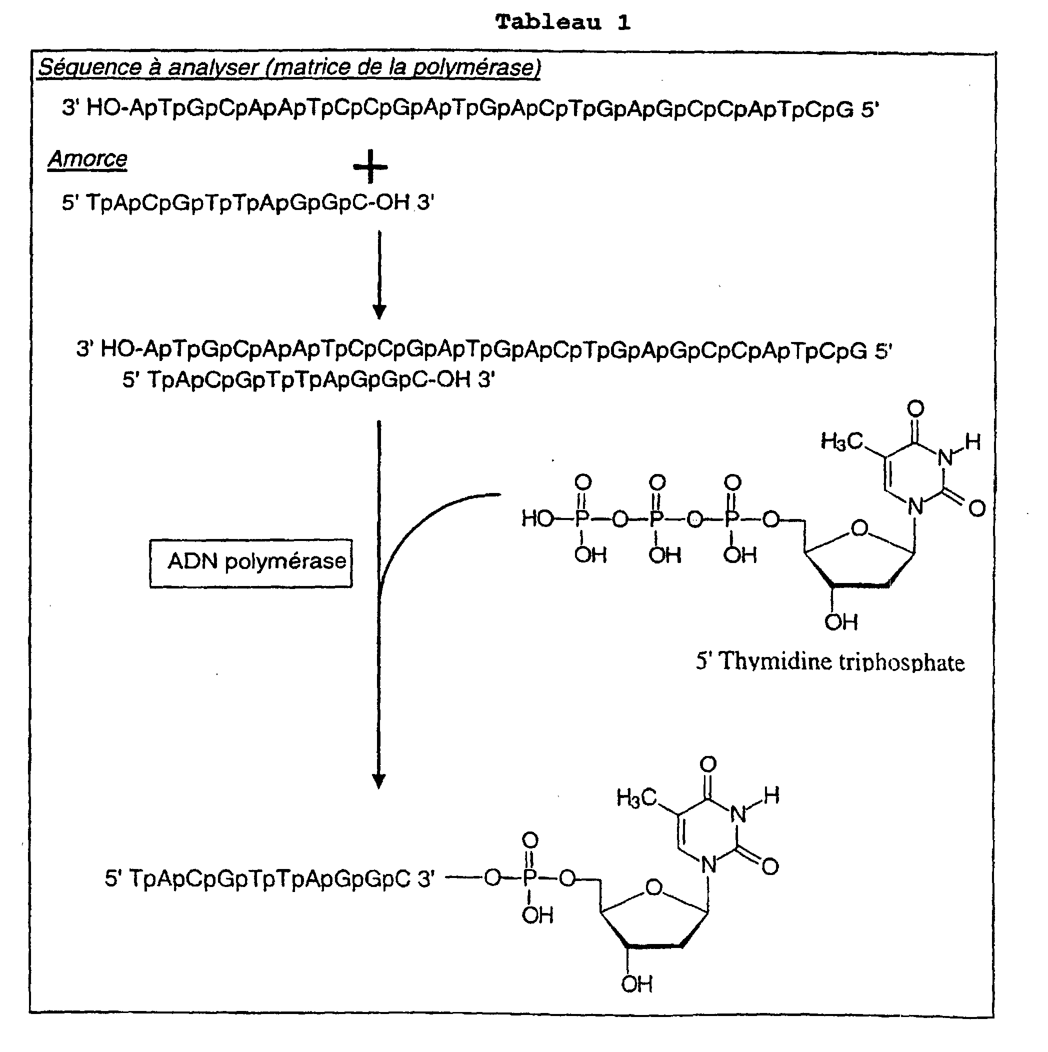

Ce tableau 1 illustre ce qui se produit lorsque l'on met en présence l'ADN polymérase, une amorce constituée par un oligonucléotide de taille réduite, généralement inférieure à 25 bases, et le mélange des quatre nucléosides triphosphates naturels avec le brin d'ADN dont on veut déterminer la séquence, qui constitue la matrice. L'amorce correspond au début de la séquence complémentaire du brin d'ADN à analyser. A partir de cette amorce, qui spontanément entre en interaction avec la séquence complémentaire du brin d'ADN à analyser (hybridation), l'enzyme incorpore des nucléotides complémentaires de la matrice pour construire par élongation-polymérisation un nouveau brin d'ADN, copie complémentaire de cette dernière. Les nouveaux nucléotides sont incorporés exclusivement à partir de l'extrémité 3'-OH terminale de la chaîne en croissance, de manière séquentielle et en respectant les règles de complémentarité entre bases de Watson & Crick. Une thymine est incorporée dans le brin néoformé par complémentarité avec une adénine présente dans le brin servant la matrice, une guanine est incorporée en complémentarité d'une cytosine et réciproquement. Si tous les composés nécessaires sont fournis en quantité non-limitante, l'enzyme catalyse la polymérisation du brin formé jusqu'à ce que ce dernier représente la totalité du brin complémentaire parfait de la matrice.

Par contre, si l'on rajoute au milieu réactionnel une molécule qui est reconnue par la polymérase mais qui ne présente pas d'extrémité 3'-OH terminale libre, chaque fois que cette molécule sera incorporée, le travail de polymérisation de l'enzyme sera interrompu parce que la chaîne ne pourra plus croître à cause de l'absence de site disponible pour ancrer un nouveau nucléotide (création de brins néoformés interrompus). C'est ce qui est illustré dans le tableau 2 qui suit avec la 3'désoxythymidine, 5'-triphosphate.

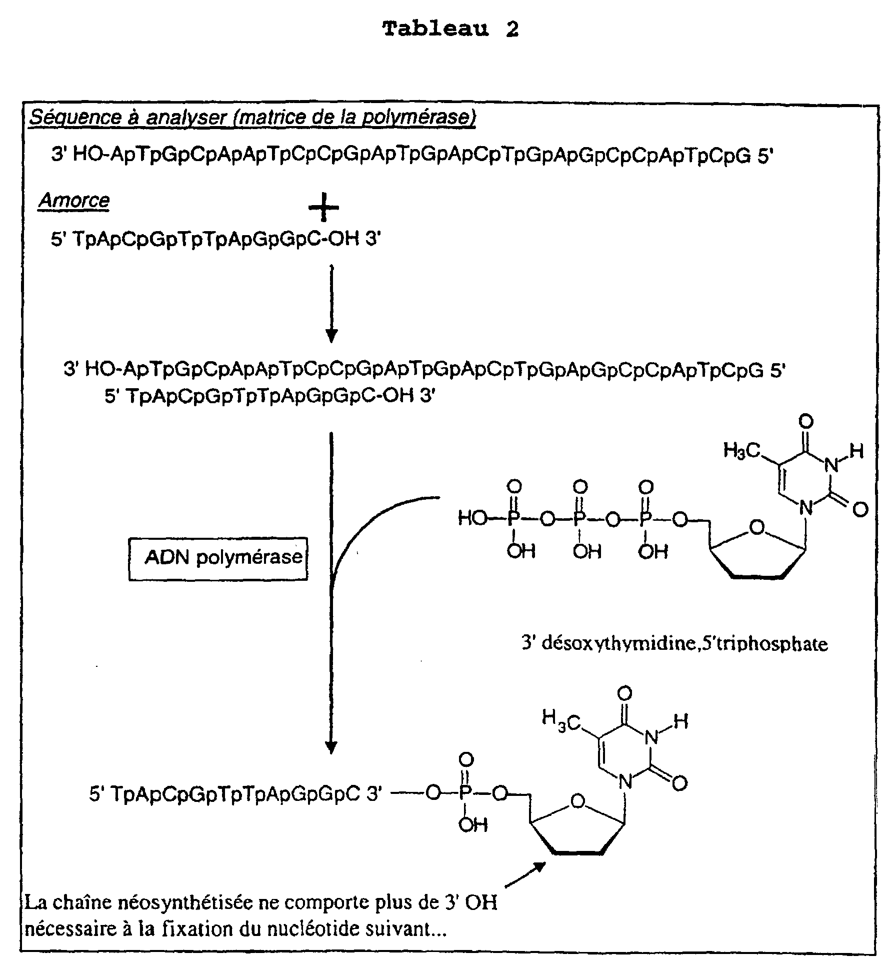

En utilisant ce dérivé de thymidine que l'on appellera « terminateur de chaîne T » à une concentration adéquate, on obtient pour une matrice donnée, une série de brins d'ADN dont la taille est fixée statistiquement par la position des adénines de la matrice. Le résultat obtenu est illustré dans le tableau 3. La séquence de la matrice est écrite dans la première ligne, la séquence des brins néoformés créés avec le terminateur de chaîne T (noté S) est écrite dans les lignes suivantes.

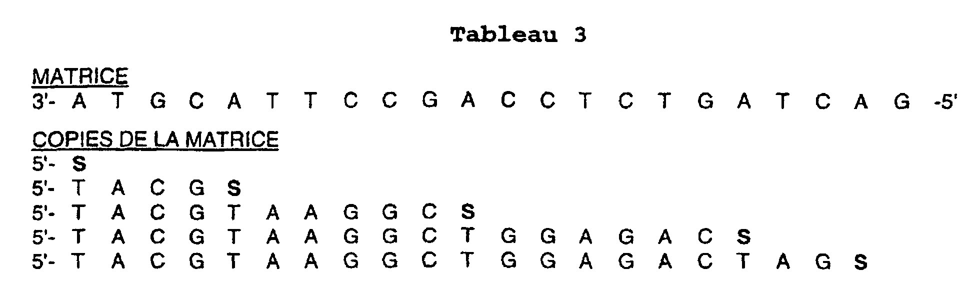

Dans cet exemple, la matrice comporte 5 adénines dans la zone qui est détaillée, l'ADN polymérase pourra donc produire 5 brins néoformés interrompus, de longueurs différentes.

Il suffit ensuite d'analyser ce mélange par électrophorèse sur gel de polyacrylamide en milieu dénaturant pour déterminer la longueur de chacun des brins obtenus en utilisant le terminateur de chaîne T. La taille des brins néoformés interrompus permet de déduire la position des adénines sur la matrice.

En recommençant trois fois cette expérience avec respectivement des produits terminateurs de chaînes A, G et C, on obtient au total quatre séries de fragments d'ADN dont la longueur permet de déterminer la séquence entière du brin matrice.

La technologie du séquençage des ARN repose sur les mêmes principes, la différence étant que l'enzyme employée est une transcriptase inverse (ou ADN polymérase-ARN dépendante).

Les produits les plus utilisés comme terminateurs de chaîne pour arrêter l'action des ADN polymérases sont des 2',3'-didésoxynucléosides triphosphates de formule : 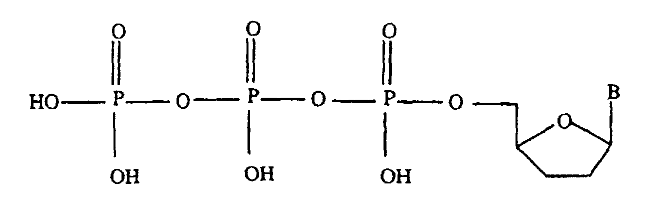 dans laquelle B représente l'une des bases nucléiques A, C, G ou T, comme il est décrit dans le document [1].

La structure de ces produits comparée à celle des nucléosides triphosphates naturels montre l'absence de la fonction hydroxyle en position 3' qui sert de position d'attache du nucléotide suivant.

La synthèse chimique des 2',3'-didésoxy-nucléotides est réalisée selon un protocole long et fastidieux comportant trois grandes étapes. Dans le cas de la guanine, la première étape de ce processus est la protection de la fonction amine exocyclique de la guanine et de la fonction hydroxyle primaire en 5' du sucre. On réalise ensuite la suppression de la fonction hydroxyle en 3', par élimination puis par réduction de la double liaison 2'-3' générée. La dernière étape est la préparation du dérivé triphosphate.

D'autres terminateurs de chaîne ont été décrits dans le document WO-A-96/23807 [2]. Ce sont les 5'-triphosphates des arabinonucléosides, des 3'-fluoro-2',3'-didésoxynucléosides, les 3'-azido-2',3'-didésoxynucléosides ou les 3'-amino-2',3'-didésoxy-nucléosides. Leur synthèse est tout aussi laborieuse.

A l'origine de la méthode de Sanger, la visualisation des fragments d'ADN synthétisés se faisait par marquage radioactif au ³²P en 5' de l'amorce utilisée pour initier la polymérisation du brin complémentaire. Une modification a été apportée en utilisant des amorces porteuses d'un fluorophore. Cette amélioration porte uniquement sur la facilité d'emploi, puisqu'elle supprime l'utilisation de matières radioactives, mais il faut toujours réaliser quatre réactions de séquençage, chacune utilisant un terminateur de polymérisation différent (terminateur A, G, T ou C).

Un nouveau pas a été franchi avec l'emploi de terminateurs de séquences porteurs de fluorophores sur leur base nucléique, comme il est décrit par Prober et al, dans Science, 238, 1987, pages 336-341 [3].

Dans ces conditions, le marquage des brins néo-synthétisés n'est plus fait avant la réaction de séquençage, mais directement au moment de l'incorporation du terminateur de séquence. En prenant soin de choisir un fluorophore présentant des propriétés optiques différentes pour chaque base de l'ADN, le protocole expérimental a été très fortement simplifié. On ne pratique plus qu'une seule réaction avec les quatre terminateurs en mélange. De ce fait, à partir d'un unique canal d'électrophorèse, on distingue les quatre nucléotides de la séquence grâce aux longueurs d'ondes d'émission différentes des quatre terminateurs.

Cette simplification dans le protocole d'analyse n'a pas que des avantages. En effet, les fluorophores sont greffés directement sur la base. Cette modification structurale, localisée au voisinage direct des sites de liaisons hydrogène régissant la reconnaissance entre les bases, entraîne une diminution de la reconnaissance par les enzymes. Pour compenser cela, une augmentation de la concentration des terminateurs est préconisée, qui conduit à une très grande consommation de la matière première ayant une très forte valeur ajoutée. De plus, la synthèse de ces molécules est toujours aussi délicate.

Le document WO 95/07907 décrit des dérivés morpholino-nucléosides dans lesquels l'hydroxyle en position 5' peut incorporer un groupement mono-, di-ou tri-phosphate. Ces dérivés comportant un cycle morpholine substitué sont utilisés pour la préparation d'anticorps dirigés contre un haptène fixé au cycle morpholine.

Le document Brown et al. : "The Reduction of the Adduct of Periodate-oxidised Adenosine-5' Phosphate and Methylamine" J. Chem. Soc., 1965, pages 5072-5073 décrit la réduction d'un dérivé morpholino-nucléoside monophosphate.

Le brevet US-A-4,515,781 concerne des oligonucléotides ayant un groupe morpholinoadenylate en chaîne terminale permettant d'augmenter la résistance aux nucléases et d'augmenter l'activité biologique de l'oligonucléotide parent.

### Exposé de l'invention

La présente invention a notamment pour objet l'utilisation, dans un procédé de séquençage de ce type, de terminateurs de chaînes constitués par des analogues de nucléosides triphosphates plus facile à synthétiser, qui permettent de plus de réaliser un marquage efficace sans modifier les bases nucléiques.

Aussi, l'invention a pour objet un procédé de séquençage d'un acide nucléique (ADN ou ARN) par la technique de polymérisation enzymatique de la séquence complémentaire de cet acide nucléique en utilisant des terminateurs de chaînes, dans lequel au moins l'un des terminateurs de chaînes a pour précurseur un composé répondant à la formule : 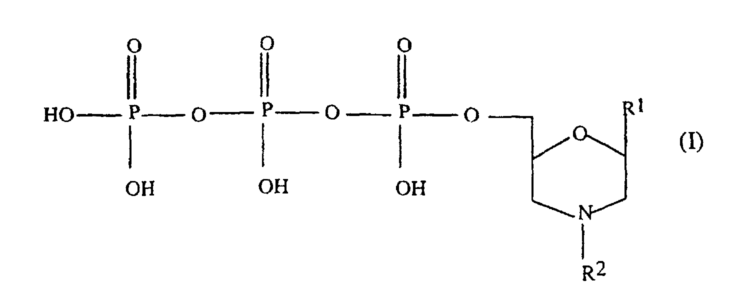 dans laquelle R¹ représente une base nucléique et R² représente un groupe répondant à l'une des formules suivantes :

-(CH₂)ₙ-NH₂ -(CH₂)ₙ-SH

- (CH₂)ₙ-COOH -(CH₂)ₙ-OH

- (CH₂)ₙ-NH-R³ -(CH₂)ₙ-SR³

- (CH₂)ₙ-CO-R³ -(CH₂)ₙ-OR³

dans lesquelles n est un nombre entier allant de 1 à 12 et R³ est un groupe dérivé d'un marqueur, d'une protéine, d'une enzyme, d'un acide gras ou d'un peptide.

Les terminateurs de chaînes utilisés dans ce procédé sont des dérivés de nucléotides comportant une base nucléique R¹ qui permet la reconnaissance par les polymérases et les transcriptases, et le respect des règles de complémentarités de Watson et Crick.

Les bases nucléiques utilisées pour R¹ peuvent être naturelles ou synthétiques. Les bases naturelles sont généralement choisies parmi l'adénine, la guanine, la cytosine, la thymine, l'uracile, la xanthine, l'hypoxanthine, la 2-aminopurine et leurs dérivés.

Les bases synthétiques sont des analogues ou des dérivés des bases nucléiques naturelles, qui sont capables d'interagir avec les bases naturelles.

De préférence, R¹ répond à l'une des formules suivantes :

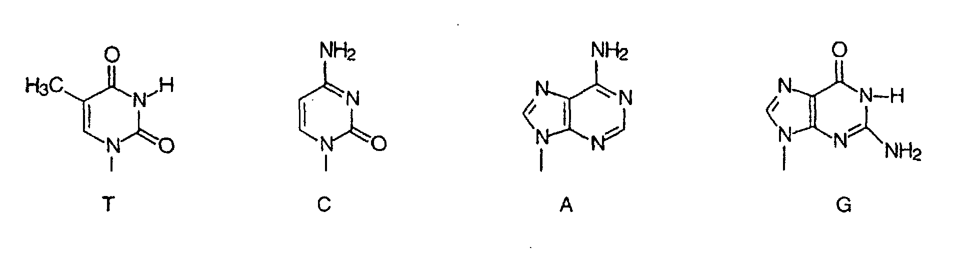

Dans les dérivés nucléotides de formule (I), la partie osidique est remplacée par une morpholine convenablement substituée comportant :
1°) Une fonction hydroxyméthyle voisine de l'oxygène cyclique, estérifiée par un groupement acide triphosphorique. Cette partie de la molécule mime la partie 4', 5' des nucléotides et permet la fixation par la polymérase ou la transcriptase à la chaîne d'ADN ou d'ARN en croissance.
2°) Une fonction amine substituée par R2, qui peut éventuellement permettre le greffage d'un chromophore ou d'un groupe biologiquement actif et surtout, qui interdit l'attachement d'un autre nucléotide (interruption de la polymérisation).

Par rapport aux dérivés classiquement utilisés dans la méthode de Sanger tels que ceux décrits dans les document [1], [2] et [3], ces composés peuvent être synthétisés en une seule étape directement à partir des ribonucléosides triphosphates, comme on le verra ci-après.

L'intérêt de ces composés réside dans le très grand choix de groupes R² (substituants du cycle morpholine) utilisables qui permettent de fonctionnaliser ce cycle. Des fonctions telles que des acides, amines, thiols ou éthers peuvent être ajoutées et permettront le greffage de composés chimiques variés, notamment de marqueurs utiles pour l'identification des fragments d'ADN ou d'ARN.

Les marqueurs utilisés pour R³ peuvent être choisis dans un ensemble très vaste de molécules connues pour le marquage de nucléotides. Ils peuvent être choisis par exemple parmi les produits radioactifs, les produits luminescents, électroluminescents et fluorescents, les molécules capables de se coupler à d'autres molécules, les molécules autorisant des interactions de type antigène-anticorps, et les marqueurs enzymatiques.

De préférence, pour le séquençage des acides nucléiques, R³ est un fluorophore, par exemple, choisi parmi tous les dérivés de la fluorescéine ou de la rhodamine. On peut aussi utiliser ceux de la biotine. En particulier, seront choisis ceux des dérivés utilisés pour le marquage des acides nucléiques.

Des dérivés de nucléosides dans lesquels la partie osidique du nucléoside a été remplacée par une morpholine, ont déjà été synthétisés dans l'art antérieur, comme il apparaît dans les documents suivants :
- Hileman et al, Bioconjugate Chemistry, 5, 1994, pages 436-444 [4],
- Broker et al, Nucleic Acids Research, 5, 1978, pages 363-385 [5],
- Agrawal et al, Nucleic Acids Research, 14, 1986, pages 6227-6245 [6],
- FR-A- 2 710 068 [7], et
- Rayford et al, Journal of Biological Chemistry, 260, 1985, pages 15708-15713, [8].

Les dérivés de nucléosides du document [4] comportent un cycle morpholino qui est substitué par une fluorescéine ou une rhodamine. Ils sont utilisés pour l'étude de protéines et non comme terminateurs de chaîne dans un procédé de séquençage d'acides nucléiques.

Leur fabrication diffère du processus que nous rapportons, car le fluorophore est incorporé directement sur le cycle morpholine. La technique que nous décrivons fait appel à une étape de purification intermédiaire qui nous a permis d'isoler et de parfaitement caractériser le produit final, au contraire de Hileman et al.

Dans le document [5], il s'agit d'ARN de transfert modifié à son extrémité 3' par un dérivé de nucléoside comportant un cycle morpholine substitué par une biotine. Ce produit est utilisé comme marqueur chimique des ARN de transfert pour étudier la localisation chromosomique des gènes des ARN de transfert.

Dans le document [6], il s'agit d'un oligonucléotide comportant un cycle morpholine couplé à une biotine, qui est utilisé comme sonde pour la détection et l'isolement de gènes spécifiques.

Le document [7] décrit des dérivés de nucléosides comportant un cycle morpholine substitué. Ils sont utilisés pour la préparation d'anticorps dirigés contre un haptène fixé au cycle morpholine du dérivé de nucléoside.

Le document [8] illustre une morpholinoadénosine substituée par CH₂COOH, utilisée pour la chromatographie d'affinité.

Ainsi, aucun de ces documents ne concerne l'utilisation de dérivés de nucléotides tels que ceux de l'invention, comme terminateurs de chaînes, dans un procédé de séquençage d'acides nucléiques selon la méthode de Sanger.

Les dérivés de nucléotides utilisés dans le procédé de l'invention, peuvent être préparés en une seule étape, directement à partir des ribonucléosides triphosphates selon le schéma réactionnel suivant illustré avec R¹ représentant l'adénine.

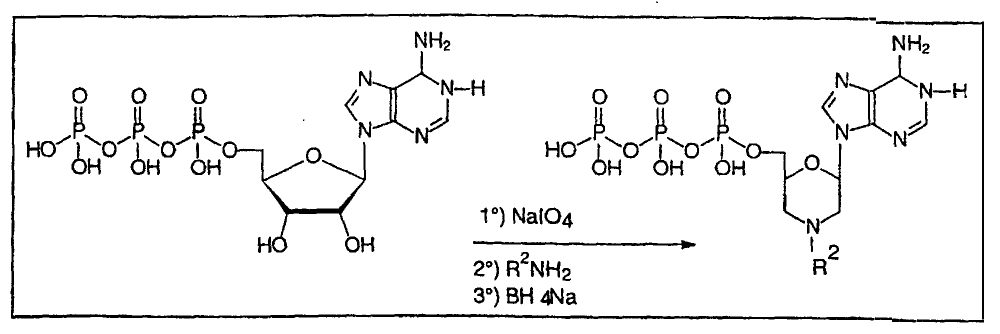

Ce procédé est du même type que les procédés décrits dans les documents [6] et [7] pour former le cycle morpholino.

On peut aussi préparer les dérivés de nucléotides de formule (I) à partir des morpholino-nucléosides et introduire ensuite le groupe triphosphate en utilisant le protocole d'Eckstein, comme il est décrit par Ludgwig et al dans J. Org. Chem. 54, 1989, pages 631-635 [9].

Les enzymes utilisables pour la polymérisation enzymatique peuvent être ceux décrits ci-dessous.

Selon l'invention, le procédé de préparation de morpholino-nucléotides de formule (I) comprend la réaction d'un nucléoside triphosphate de formule : 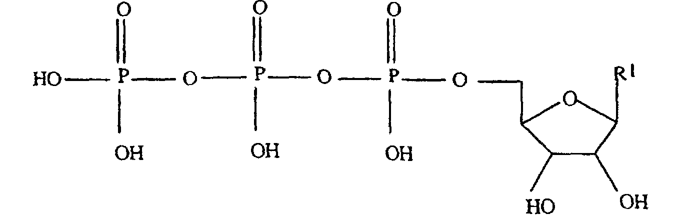 dans laquelle R¹ a la signification donnée ci-dessus, avec: un periodate, un composé de formule R² NH₂ dans laquelle R² a la signification donnée ci-dessus et du borohydrure de sodium.

L'invention concerne également l'utilisation d'un dérivé de nucléotide ayant pour précurseur un composé de formule (I) pour le marquage en 3' de fragments d'acide nucléique (ADN ou ARN) par incorporation enzymatique du dérivé de nucléotide à l'extrémité 3'OH du fragment d'acide nucléique.

Elle concerne aussi le procédé de frabrication d'un fragment d'acide nucléique (ADN ou ARN) marqué 3' par incorporation enzymatique du dérivé de nucléotide mentionné ci-dessus à l'extrémité 3'OH du fragment d'acide nucléique.

L'enzyme utilisée peut être le fragment de Klenow de l'ADN polymérase, et on utilise alors une matrice ou template pour fixer le morpholino-nucléoside sur le fragment d'acide nucléique qui sert d'amorce.

L'enzyme utilisée peut aussi être une polymérase thermorésistante d'une bactérie thermophile ou la terminal transférase ou la transcriptase inverse.

Les fragments d'ADN ou d'ARN ainsi marqués sont utilisables pour bloquer toute ligation ultérieure et assurer une protection contre les exonucléases, ainsi que pour détecter des fragments d'ADN ou d'ARN.

On peut encore utiliser un morpholino-nucléotide modifié ayant pour précurseur un composé de formule (I) pour modifier un fragment d'acide nucléique (ADN ou ARN) par incorporation enzymatique à l'extrémité 3' de celui-ci d'un morpholino-nucléotide modifié ayant pour précurseur un composé de formule (I) comprenant en R³ un composé choisi parmi les photoréticulants par exemple pour réticulation sur ADN ou sur un support quelconque ; les acides gras, les peptides hydrophobes, les anticorps, par exemple pour faciliter la pénétration dans les cellules, les enzymes ou parties d'enzymes telles que les phosphatases alcalines, les peroxydases, les acétylcholinestérases pour la détection, les enzymes de restriction pour le clivage de l'ADN vicinal, et les fluorophores.

Comme précédemment l'incorporation de ce morpholino-nucléotide modifié est effectuée par voie enzymatique. Les bases azotées, les marqueurs et les enzymes utilisables peuvent être les mêmes que ceux précités.

Selon l'invention, le dérivé de nucléotide, le morpholino-nucléotide modifié et le terminateur de chaîne utilisés respectivement pour le marquage en 3' de fragments d'acide nucléique, pour la modification de fragments d'acide nucléique ou pour le séquençage d'un acide nucléique, peuvent être le composé (I) sous forme de monophosphate.

D'autres caractéristiques et avantages de l'invention apparaîtront mieux à la lecture de la description qui suit d'exemples de réalisation, donnés bien entendu à titre illustratif et non limitatif, en référence au dessin annexé.

### Brève description des figures

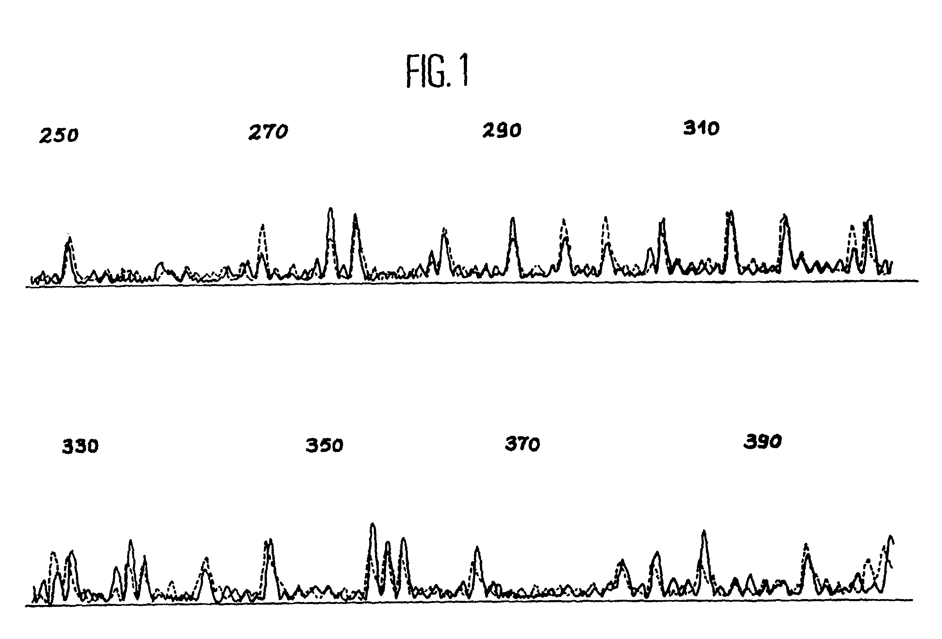

La figure 1 est un diagramme illustrant les résultats obtenus pour le séquençage d'ADN plasmidique avec le terminateur de chaîne de l'invention (courbe en trait plein) et avec le terminateur de chaîne de l'art; antérieur (courbe en tirets).

La figure 2 est un diagramme illustrant les résultats obtenus en testant les morpholino A putrescine (MATPP) et morpholino A fluorescéine (MATPPF) en séquençage.

La figure 3 est un schéma illustrant le résultat sur gel de polyacrylamide d'un test permettant de suivre l'allongement d'un oligonucléotide A et l'incorporation de morpholino A putrescine.

### Exposé détaillé des modes de réalisation

Les exemples 1 à 4 qui suivent, illustrent la synthèse de morpholino-nucléotides de formule (I).

### Exemple 1 : Synthèse de la 4-(carboxyméthyl)-2-(adénosin-9-yl)-6-(hydroxyméthyl)morpholine-6-triphosphate (morpholino A glycine) 1.

Cette morpholino A glycine 1 répond à la formule (I) dans laquelle R¹ est l'adénine et R² est le groupe -CH₂-COOH.

Dans cet exemple, toutes les réactions sont conduites à la température ambiante, sous agitation magnétique, dans un ballon de 50 mL.

On dissout 1,000 g, (1,8 mmol, 1 éq.) de 5'-adénosine triphosphate dans 10 mL d'eau, puis on ajoute 1 éq. de periodate de sodium (388 mg, 1,8 mmol). La solution est alors agitée pendant 35 minutes.

La glycine (682 mg, 9,1 mmol, 5 éq.) en solution dans 2 mL d'eau (pH = 9,5-10) est ajoutée et l'on remonte le pH de la solution à 9,5-10 avec du carbonate de potassium solide. La solution est agitée pendant 55 minutes. Le mélange réactionnel jaunit.

Du borohydrure de sodium (au total 166 mg, 4,4 mmol, 2,5 éq.) est ajouté en six fractions équivalentes, chacune dissoute dans 0,2 mL d'eau. Après ajout de la première, on note un dégagement gazeux. Les autres fractions, chacune dissoute juste avant ajout, sont additionnées toutes les heures.

Après une nuit, la solution est neutralisée par ajout d'acide formique 1M jusqu'à pH 4-5, puis elle est évaporée.

Une analyse par chromatographie en polarité de phase inversée sur une colonne Merck LiChrocart 125-4 LiChrospher 100 RP-18 ("endcapped", 5 µm, 125x4 mm) en utilisant un débit de 1 mL/min et l'éluant acétate de triéthylammonium TEAA 25mM/méthanol MeOH [98/2], indique un rendement de 40 % (k' = 3,85).
Purification : elle est faite par chromatographie liquide à haute performance (HPLC) préparative en utilisant une colonne Macherey Nagel Nucléosil 7 C-18 7 µm, 250x21 mm) avec un débit de 8 mL/min et du bicarbonate de triéthylammonium TEAB 25mM, comme éluant.
Caractérisation :
- RMN ¹H : δ (ppm) : 8,47 (s, 1H, H2), 8,37 (s, 1H, H8), 6,26 (dd, 1H, H1'), 4,54(m, 1H, H4'), 4,28 (m, 1H, H5"), 4,22 (m, 1H, H5'), 3,70 (m, 1H, H2'), 3,68 (s, 2H, CH₂-Glycine), 3,41 (m, 1H, H2"), 3,45 (m, 1H, H3'), 3,30 (m, 1H, H3"),
- RMN ¹³C : δ (ppm) : 152,7 (C2), 140,5 (C8), 78,6 (C1'), 74,1 (C4'), 66,4 (C5'), 60,6 (CH₂), 54,5 (C2'), 53,6 (C3')
- RMN ³¹P : δ (ppm) : -6,44 (d, 1P, γP), -11,68 (d, 1P, αP), -22,11 (t, 1P, βP)
- Spectrométrie de masse : M-H⁻ = 547,04 g.mol⁻¹

### Exemple 2 : Synthèse de la 4-(carboxyméthyl)-2-(thymidin-1-yl)-6-(hydroxyméthyl)morpholine-6-triphosphate (morpholinoT glycine) 4.

Ce composé **4** répond à la formule (I) avec R¹ représentant la thymine et R² représentant le groupe -CH₂-COOH.

Dans cet exemple, on prépare tout d'abord le morpholino-nucléoside, puis on le transforme en triphosphate.

### a) Préparation du morpholino-nucléoside de la ribothymidine 2.

Toutes les réactions sont conduites à la température ambiante, sous agitation magnétique, dans un ballon de 250 mL.

La ribothymidine (3,500 g, 13,5 mmol, 1 éq) est dissoute dans 35 mL d'eau, puis est additionnée de 1 éq. de periodate de sodium (2,900 g, 13,5 mmol). La solution est alors agitée pendant 45 minutes.

La glycine (5,089 g, 67,8 mmol, 5 éq) dans 35 mL d'eau (pH = 9,5-10) est ajoutée et le pH de la solution est remonté à 9,5-10 avec du carbonate de potassium. La solution est agitée pendant une heure et 45 minutes. Le mélange réactionnel jaunit.

Un sixième de borohydrure de sodium (au total 1,280 g, 33,8 mmol, 2,5 éq) dissous dans 3,5 mL d'eau est ajouté à la solution. On note un dégagement gazeux. Les autres sixièmes, chacun dissous juste avant ajout, sont additionnés toutes les heures.

Après une nuit, la solution est neutralisée par ajout d'acide formique 1M jusqu'à pH4-5, puis elle est évaporée.

Une analyse par chromatographie en polarité de phase inversée sur colonne Merck LiChrocart 125-4 LiChrospher 100 RP-18 ("endcapped", 5µm, 125x4 mm), avec un débit de 1 mL/min, en utilisant comme éluant : TEAA 25mM/MeOH [99/1], indique un rendement de 32% (k'=8,83).
Purification : elle est faite par chromatographie "flash" sur une colonne de silice à polarité de phase inversée C-18 (Matrex, Amicon). L'éluant est de l'eau.
Caractérisation :
- RMN ¹H : δ (ppm) : 7,77 (s, 1H, H6), 5,92 (dd, 1H, H1'), 4,07(m, 1H, H4'), 3,77 (m, 2H, H5', H5"), 3,22 (s, 2H, CH₂ glycine), 3,13 (dd, 1H, H2"), 2,99 (dd, 1H, H3"), 2,51 (t, 1H, H2'), 2,34 (t, 1H, H3'), 1,98 (s, 3H, CH₃ base).

### b) Préparation du morpholino-nucléoside monophosphate de la ribothymidine 3.

A 342 mg d'imidazole (5,0 mmol, 3 éq) séchés au dessicateur puis repris dans 5 mL de pyridine rigoureusement anhydre, sont additionnés 234 µL d'oxychlorure trichlorure de phosphore (2,5 mmol, 1,5 éq.). Le mélange est placé sous agitation pendant 30 minutes sous air sec.

Parallèlement, 500 mg de la morpholinothymidine (1,7 mmol, 1 éq.) obtenue en a) sont séchés 3 fois dans la pyridine, puis repris dans 5 mL de pyridine anhydre.

Le mélange imidazole/POCl₃/pyridine sous argon est additionné à la solution de morpholinonucléoside et l'ensemble est placé sous agitation pendant 48 heures à température ambiante. Puis 100 µL d'eau sont ajoutés en prenant soin de refroidir le ballon réactionnel dans un bain de glace. Le mélange réactionnel est évaporé à sec puis repris 2 fois avec de l'eau et évaporé afin d'éliminer la pyridine.

Une analyse par chromatographie en polarité de phase inversée sur colonne Macherey Nagel Nucléosil 5 C-18 (7 µm, 120x3 mm), à un débit de ; 1 mL/min, en utilisant comme éluant : TEAA 25mM-MeOH [97/3], indique un rendement de 33% (k'=0,62).
Purification : elle est faite par HPLC préparative sur H : colonne Macherey Nagel Nucléosil 7 C-18 (7 µm, 250x21 mm) à un débit de 5 mL/min en utilisant l'eau comme éluant.
Caractérisation :
- RNN ¹H : δ (ppm) : 7,80 (s, 1H, H6), 5,95 (dd, 1H, H1') 4,19(m, 1H, H4'), 3,94 (t, 2H, H5', H5"), 3,28 (s, 2H, CH₂ glycine), 3,24 (m, 1H, H2"), 3,10 (m, 1H, H3"), 2,53 (t, 1H, H2'), 2,39 (t, 1H, H3'), 2,00 (s, 3H, CH₃ base)
- RMN ³¹P : δ (ppm) : 1,74 (s)

### c) Préparation du morpholino-nucléoside triphosphate de la ribothymidine 4.

1,097 g de carbonyldiimidazole (6,7 mmol, 5 éq.) dissous dans 5 mL de diméthylformamide anhydre sont additionnés au sel de tributylammonium du morpholinonucléoside monophosphate de la thymine 3 obtenue en b) (511 mg, 1,3 mmol, 1 éq.) dissous dans 3 mL de diméthylformamide anhydre. Le mélange est placé sous agitation à température ambiante pendant cinq heures. L'excès de carbonyldiimidazole est détruit par ajout de 436 µL de méthanol (10,8 mmol, 8 éq.). Après 30 minutes, 5 équivalents de pyrophosphate de tributylammonium (3,008 g, 6,7 mmol) en solution dans 5 mL de diméthylformamide sont ajoutés. Le mélange est placé sous agitation pendant deux jours, puis le mélange réactionnel est filtré et évaporé à sec.

Une analyse par chromatographie en polarité de phase inversée est effectué sur une colonne SFCC PVDI 31 (5 µm, 100x4,6 mm), à un débit de 1 mL/min, en utilisant comme éluant un gradient de formiate d'ammonium (FA), dans les conditions suivantes :

| t(min) | FA 25 mM | FA 0,9 M |
|---|---|---|
| | (%) | (%) |
| 0 | 100 | 0 |
| 10 | 100 | 0 |
| 40 | 0 | 100 |
| 41 | 0 | 100 |
| 43 | 100 | 0 |

Ceci indique un rendement de 27% (k'=13,84).
Purification : elle est faite par chromatographie "flash" sur une colonne de phase échangeuse d'ions (DEAE Sepharose Fast Flow, Pharmacia Biotech). L'éluant est un gradient de TEAB (de 25 mM à 0,9 M).
Caractérisation :
- RMN ¹H : δ (ppm) : 7,74 (s, 1H, H6), 5,92 (dd, 1H, H1'), 4,25(m, 1H, H4'), 4,15 (m, 2H, H5', H5"), 3,81 (s, 2H, CH₂ glycine), 3,54 (d, 1H, H2"), 3,10 (t, 1H, H3"), 2,56 (t, 1H, H2'), 2,45 (t, 1H, H3'), 1,95 (s, 3H, CH₃ base)
- RMN ³¹P : δ (ppm) : -10,03 (d, 1P, γP), -10,88 (d, 1P, αP), -22,65 (t, 1P, βP)
- Spectrométrie de masse : M-H⁻ = 540,41 g.mol⁻¹

### Exemple 3 : Synthèse de la 4-(carboxyméthyl-2-(guanin-9-yl)-6-(hydroxyméthyl) morpholine-6-triphosphate (morpholino G glycine) 5.

Cette morpholino G glycine **5** répond à la formule (I) avec R¹ = guanine et R² = -CH₂COOH.

La guanosine,5'4riphosphate (50 mg, 0,08 mmol, 1 éq.) est dissoute dans 2 mL d'eau, puis est additionnée de 1 éq. de periodate de sodium (18 mg, 0,08 mmol). La solution est alors agitée pendant 35 minutes. La glycine (31 mg, 0,42 mmol, 5 éq.) en solution dans 2 mL (pH = 9,5-10) est ajoutée et l'on remonte le pH de la solution à 9,5-10 avec du carbonate de potassium solide (contrôle avec du papier pH). La solution est agitée pendant 45 minutes. Du borohydrure de sodium (au total 8 mg, 0,21 mmol, 2,5 éq.) est ajouté en six fractions équivalentes, chacune dissoute dans 0,1 mL d'eau. Les autres fractions, chacune dissoute juste avant ajout, sont additionnées toutes les heures. Après une nuit, la solution est neutralisée par ajout d'acide formique 1M jusqu'à pH 4-5, puis elle est évaporée.

Une analyse par chromatographie en polarité de phase inversée (Système E) sur colonne SFCC PVDI 31 (5 µm, 100 x 4,6 mm) avec un débit : 1 mL/min, en utilisant comme éluant un gradient de formiate d'ammonium, dans les conditions suivantes :

| t(min) | FA 25 mM | FA 1 M |
|---|---|---|
| | (%) | (%) |
| 0 | 100 | 0 |
| 3 | 100 | 0 |
| 10 | 0 | 100 |
| 15 | 0 | 100 |
| 17 | 100 | 0 |

Cette analyse donne un rendement de 39 % (k'=5,5).

On purifie le composé **5** par HPLC préparative en utilisant le système F : Colonne Vydac Sax-Protéin (8 µm) 100 x 4,6 mm). Débit : 10 mL/min. Eluant : gradient de formiate d'ammonium, dans les conditions suivantes :

| t(min) | FA 25 mM | FA 1 M |
|---|---|---|
| | (%) | (%) |
| 0 | 100 | 0 |
| 3 | 100 | 0 |
| 10 | 0 | 100 |
| 15 | 0 | 100 |
| 17 | 100 | 0 |

On obtient 14 mg du composé **5**, soit 26,1 % de rendement.
Caractérisation :
- RMN ¹H (AM 400 Brüker) : δ (ppm) : 8,07 (s, 1H, H8) ; 6,06 (dd, 1H1, H1') 4,51 (m, 1H, H4') ; 4,22 (m, 2H, H5', H5") ; 3,71 (m, 1H, H2") ; 3,67 (s, 2H, -CH₂ glycine) ; 3,46 (m, 1H, H3") ; 3,38 (m, 1H, H2') ; 2,95 (m, 1H, H3').
- RMN ¹³C (AM 400 Brüker) : δ (ppm) : 173,50 (-COOH); 158,91 (C6) ; 153,98 (C2); 151,07 (C4); 137,39 (C8); 115,94 (C5) ; 77,87 (C 1') ; 73,62 (C4') ; 65, 61 (C5') ; 59,98 (-CH₂-) ; 53,28 (C2') ; 51,88 (C3').
- RMN ³¹P (U 400 Varian) : δ (ppm) : -7,14 (d, 1P, γP) ; 8,68 (d, 1P, αP) ; -20,28 (t, 1P, βP).
- Spectrométrie de masse (appareil LCQ en mode positif) : M+H⁺ = 564,9 g.mol⁻¹.
- Spectre UV : λmax =256 nm.
- Electrophorèse capillaire :
   µep = -4,28 x 10⁻⁴ cm².V⁻¹.s⁻¹.

### Exemple 4 : Synthèse de la 4-(carboxyméthyl)-2-(cytosin-1-yl)-6-(hydroxyméthyl) morpholine-6-triphosphate (morpholino C glycine) 6.

Le composé **6** répond à la formule (I) avec R¹ = cytosine et R² = -CH₂-COOH.

Toutes les réactions sont conduites à température ambiante, sous agitation magnétique, dans un ballon de 20 mL.

La réaction est la même que pour le composé 5 partir de la 5'-cytosine triphosphate (50,0 mg, 0,09 mmol, 1 éq.), de periodate de sodium (21 mg, 0,09 mmol, 1 éq.), de la glycine (36 mg, 0,48 mmol, 5 éq.) en solution dans 2 mL d'eau (pH = 9,5-10), du borohydrure de sodium (au total 9 mg, 0,23 mmol, 2,5 éq.), ajouté en six fractions équivalentes, chacune dissoute dans 0,05 mL d'eau.

Une analyse par chromatographie sur colonne de phase échangeuse d'ions (système E) comme dans l'exemple 3, indique un facteur de capacité k'=4,08.

On purifie le produit par HPLC semi-préparative en utilisant le système F comme dans l'exemple 3.

17 mg de produit sont isolés, ce qui correspond à 24,3 % de rendement.
Caractérisation :
- RMN ¹H (AM 400 Brüker) : δ (ppm) : 7,93 (d, 1H, H6) ; 6,25 (dd, 1H, H1') ; 6,20 (d, 1H, H5) ; 4,51 (m, 1H, H4') ; 4,27 (m, 2H, H5', H5") ; 3,85 (m, 4H, H2" + H3" + -CH₂ glycine) ; 3,33 (t, 1H, H2') ; 3,22 (t, 1H, H3').
- RMN ¹³C (AM 400 Brüker) : δ (ppm) : 173,O5 (-COOH) ; 165,13 (C4) ; 154,23 (C2) ; 140,93 (C6) ; 95,48 (C5) ; 80,42 (C1') ; 78,44 (C4') ; 69,37(C5") ; 64,57 (-CH₂-) ; 54,66 (C2') ; 53,67 (C3').
- RMN ³¹P (WM 250 srüker) : δ (ppm) : -7,99 (d, 1P, γP) ; -10, 10 (d, 1P, αP) ; -21,28 (t, 1P, βP).
- Spectrométrie de masse (appareil VG ZAB-2-EQ, mode négatif) :
   M - H⁻ = 521,9 g.mol⁻¹.
- Spectre UV : λmax = 270 nm
- Electrophorèse capillaire :
   µep = -4,28 x 10⁻⁴ cm².V⁻¹.s⁻¹.

### Exemple 5 : Synthèse de la 4-(aminobutyl)-2-(adénosin-9-yl)-5-(hydroxyméthyl)morpholine-6-triphosphate (morpholino A putrescine) 7.

Cette morpholino A putrescine 7 répond à la formule (I) avec R¹ représentant l'adénine et R² représentant le groupe -(CH₂)₄-NH₂.

Toutes les réactions sont conduites à la température ambiante, sous agitation magnétique, dans un ballon de 100 mL.

La 5'-adénosine triphosphate (500 mg, 0,9 mmol, 1 éq.) est dissoute dans 10 mL d'eau, puis est additionnée de 1 éq. de periodate de sodium (194 mg, 0,9 mmol). La solution est alors agitée pendant 45 minutes.

La putrescine (456µL, 4,5 mmol, 5 éq.) est ajoutée. La solution est agitée pendant 45 minutes. Le mélange réactionnel jaunit.

Un sixième de borohydrure de..sodium (au total 86 mg, 2,3 mmol, 2,5 éq.) dissous dans 0,1 mL d'eau est ajouté à la solution. On note un dégagement gazeux. Les autres sixièmes, chacun dissous juste avant ajout, sont additionnés toutes les heures.

Après une nuit, la solution est neutralisée par ajout d'acide formique 1M jusqu'à pH4-5, puis elle est évaporée.

On effectue une analyse par chromatographie en polarité de phase inversée sur colonne Merck LiChrocart 125-4 LiChrospher 100 RP-18 ("endcapped", 5 µm, 125x4mm) avec un débit de 1 mL/min, en utilisant comme éluant un gradient TEAB 25mM/MeOH, dans les conditions suivantes :

| t(min) | TEAB | MeOH |
|---|---|---|
| | (%) | (%) |
| 0 | 97 | 3 |
| 2 | 97 | 3 |
| 10 | 90 | 10 |
| 15 | 90 | 10 |
| 17 | 97 | 3 |

Cette analyse indique un rendement de 67 % (k'=3,81).

On purifie le produit 7 par HPLC semi-préparative sur la colonne Phenomenex Ultremex 5-C18 (250x10 mm) avec un débit de 4 mL/min, et en utilisant comme éluant un gradient TEAB 25 mM/MeOH, dans les conditions suivantes :

| t(min) | TEAB | MeOH |
|---|---|---|
| | (%) | (%) |
| 0 | 95 | 5 |
| 3 | 95 | 5 |
| 8 | 90 | 10 |
| 10 | 95 | 5 |

Caractérisation :
- RMN ¹H : δ (ppm) : 8,44 (s, 1H, H2), 8,33 (s, 1H, H8), 6,06 (dd, 1H, H1'), 4,35(m, 1H, H4'), 4,22 (m, 2H, H5', H5"), 3,39 (d, 1H, H2'), 3,22 (t, 1H, H3"), 3,14 (s, 2H, CH₂ putrescine), 2,92 (t, 1H, H2'), 2,74 (s, 2H, CH₂ putrescine), 2,54 (t, 1H, H3'), 1,78 (s, 4H, (CH₂)₂ putrescine).
- RMN ³¹P : δ (ppm) : -8,45 (dd, 1P, γP),
- 13,25 (dd, 1P, αP), -24,20 (t, 1P, βP)
- Spectrométrie de masse : M+H⁺ = 561,92 g.mol⁻¹

### Exemple 6 : Synthèse de la 4-(aminobutyl)-2-(thymidin-1-yl)-6-(hydroxyméthyl) morpholine-6-triphosphate (morpholino T putrescine) 9.

Le composé **9** répond à la formule (I) avec R¹ = thymine et R² = -(CH₂)₄-NH₂.

### a) Préparation de la 4-(aminobutyl)-2-(thymidin-1-yl)-6-(hydroxyméthyl) morholine-6-hydroxyle 8.

Toutes les réactions sont conduites à température ambiante, sous agitation magnétique, dans un ballon de 250 mL.

La ribothymidine (2,000 g, 7,74 mmol, 1 éq.), est dissoute dans 30 ml d'eau, puis est additionnée de 1 éq. (1,656 g, 7,75 mmol) de periodate de sodium. La solution est alors agitée pendant 70 minutes. La putrescine (3,9 ml, 38,75 mmol, 5 éq.) est ajoutée. La solution est agitée pendant 50 minutes. Le mélange réactionnel jaunit.

Un sixième de borohydrure de sodium (au total 735 mg, 19,42 mmol, 2,5 éq.) dissous dans 0,25 mL d'eau est ajouté à la solution. On note un dégagement gazeux. Les autres sixièmes, chacun dissous juste avant ajout dans 0,25 mL d'eau sont additionnés toutes les heures. Après une nuit, la solution est neutralisée par ajout d'acide formique 1M jusqu'à pH 4-5, puis elle est évaporée.

On effectue une analyse par chromatographie en polarité de phase inversée en utilisant le système G : colonne Merck-LiChrocart 125-4 LiChrospher 100 RP-18 (« endcapped », 5 µm, 125 x 4 mm). Débit : 1 mL/min. Eluant : gradient TEAB 25 Mm/CH₃CN, dans les conditions suivantes :

| t(min) | TEAB 25 mM | CH₃CN |
|---|---|---|
| | (%) | (%) |
| 0 | 100 | 0 |
| 4 | 100 | 0 |
| 15 | 85 | 15 |
| 18 | 100 | 0 |

Ceci indique un rendement de 76 % (k' =5,7).

On purifie par HPLC préparative en utilisant le système H : colonne Macherey Nagel Nucléosil 7 C-18 (7 µm, 250 x 21 mm). Débit : 10 mL/min. Eluant : TEAB 25 mM / CH3CN [85/15].

On obtient 1,56 g du composé 8, soit 64,6 % de rendement.
Caractérisation :
- RMN 1H (AC 200 Brüker) : δ (ppm) : 7,69 (s, 1H, H6) ; 5,88 (dd, 1H, H1') ; 4,01 (m, 1H, H4') ; 3,80 (m, 1H, H5', H5") ; 3,08 (m, 4H, H2", H3", 2Ha) ; 2,63 (m, 2H, 2 Hd) 2,33 (t, 1H, H2') ; 2,22 (t, 1H, H3') ; 1,98 (m, 3H, -CH3); 1,74 (m, 4H, 2 Hb, 2 Hc).
- RMN 13C (AC 200 Brüker) : δ (ppm): 171,16 (C2) ; 154,58 (C4) ; 135,93 (C6); 110,46 (C5) ; 78,62 (C11) ; 75,04 (C4') ; 61,10 (C5') ; 55,82 (C3') ; 53,49 (C2') ; 51,30 (Ca) ; 38,39 (Cd) ; 24,50 (Cc) ; 21,31 (Cb) ; 11,10 (-CH₃).
- Spectre UV : λmax =266 nm.

### b) Préparation de la 4-(aminobutyl)-2-(thymidin-1-yl)-6-(hydroxyméthyl) morpholine-6-triphosphate 9.

La morpholinothymidine/putrescine **8** (249 mg, 0,80 mol, 1 éq.) est séchée à la pompe à palette pendant 1 heure. On ajoute ensuite 256 mg de Proton-sponge® (1,19 mmol, 1,5 éq.)et l'on additionne 2 mL de triméthylphosphate anhydre ; on place le milieu dans un bain de glace, sous agitation, puis on additionne 109 µL d'oxychlorure de phosphore (au total 2,24 mmol, 2,8 éq.). Après 2 h 30, on additionne à nouveau 50 mL d'oxychlorure de phosphore, et l'on recommence 12 h après. Puis, on ajoute 8 mL d'une solution 0,5 M de pyrophosphate sous forme de sel de tributylammonium (4,0 mmol, 5 éq.), dans le DMF anhydre. On laisse sous agitation à 0°C pendant une minute, puis le milieu est séché au rotavapeur et pompe à palette.

Une analyse par chromatographie en polarité de phase inversée en utilisant le système I : colonne Vydac Sax-Protein (8 µm, 100 x 4,6 mm) avec un débit : 10 mL/min en utilisant comme éluant un gradient de formiate d'ammonium, dans les conditions suivantes :

| t(min) | FA 25 mM | FA 1M |
|---|---|---|
| | (%) | (%) |
| 0 | 100 | 0 |
| 1 | 100 | 0 |
| 15 | 70 | 30 |
| 17 | 100 | 0 |

Elle indique un facteur de capacité k'= 3,2

On purifie par HPLC préparative en utilisant le système I décrit ci-dessus.

On obtient 48 mg de **9**, soit 13,2 % de rendement.
Caractérisation :
- RMN ¹H (AM 400 Brüker) : δ (ppm) : 7,83 (s, 1H, H6) ; 6,31 (dd, 1H, H1') ; 4,68 (m, 1H, H4') ; 4,39 (m, 1H, H5', H5") ; 4,01 (d, 1H, H2") ; 3,93 (d, 1H, H3") ; 3,58 (m, 2H, 2 Ha) ; 3,51 (t, 1H, H2') ; 3,41 (m, 1H, H3') ; 3,28 (m, 2H, 2 Hd) ; 2,10 (s, 5H, -CH₃ + 2 Hb) ; 2,00 (m, 2H, 2Hc).
- RMN ¹³C (Am 400 Bruker) : δ (ppm) : 166,36 (C2) ; 151,03 (C4); 136,73 (C6) ; 112,42 (C5) ; 77,33 (C1') ; 72,46 (C4') ; 65,10 (C5') ; 57,04 (C3) ; 51,71 (C2') ; 51,13 (Ca) ; 98,91 (Cd); 23,85 (Cc) ; 20,50 (Cb) ; 11,62 (-CH₃).
- RMN ³¹P (U 400 Varian) : δ (ppm) : -8,19 (s, 2P, γP, αP) ; -18,99 (t, 1P, βP).
- Spectrométrie de masse (appareil LCQ en mode négatif) : M - H⁻ = 551,3 g.mol⁻¹.
- Spectre UV : λmax =262 nm.
- Electrophorèse capillaire :
   µep = -4,69 x 10⁻⁴ cm².V⁻¹.s⁻¹.

### Exemple 7 : Synthèse de la 4-(aminobutyl)-2-(guanosin-9-yl)-6-(hydroxyméthyl morpholine-6-triphosphate (morpholino G putrescine) 10.

Le composé **10** répond à la formule (I) avec R¹ = guanine et R² = -CH₂)₄-NH₂-.

Toutes les réactions sont conduites à température ambiante, sous agitation magnétique, dans un ballon de 50 mL.

La guanosine,5'-triphosphate (50 mg, 0,17 mmol, 1 éq.) est dissoute dans 5 mL d'eau, puis est additionnée de 1 éq. de periodate de sodium (37 mg, 0,17 mmol, 1 éq.). La solution est alors agitée pendant 30 minutes.

La putrescine (85 µL, 0,84 mmol, 5 éq.) est ajoutée et l'on mesure le pH de la solution qui est égal à la. Si une valeur inférieure avait été trouvée, on aurait ajouté du carbonate de potassium pour obtenir cette valeur. La solution est agitée pendant 45 minutes.

Du borohydrure de sodium (au total 8,7 mg, 0,45 mmol, 2,5 éq.) est ajouté en six fractions équivalentes, chacune dissoute dans 0,1 mL d'eau. Les autres fractions, chacune dissoute juste avant ajout, sont additionnées toutes les heures.

Après une nuit, la solution est neutralisée par ajout d'acide formique 1M jusqu'à pH 4-5, puis elle est évaporée.

On purifie le composé 10 par précipitation au méthanol, puis passage sur 5 mL de résine Dowex sous forme Na+.

On obtient 68 mg de composé 10, soit un rendement de 62,2 %.
Caractérisation :
- RMN ¹H (AM 400 Brüker) : δ (ppm) : 8,29 (s, 1H, H8) ; 6,31(dd, 1H, H1') ; 4,74 ((m, 1H, H4') ; 4,37 (m, 2H, H5', H5") ; 3,99 (m, 1H, H2") ; 3,96 (m, 1H, H3") ; 3,79 (t, 1H, H2'); 3,47 (m, 2H, 2 Hb) ; 3,39 (t, 1H, H3') ; 3,19 (m, 2H, 2 Hc) ; 2,06 (m, 2H, 2 Ha) ; 1,91 (m, 2H, 2 Hd).
- RMN ¹³C (AM 400 Brüker) : δ (ppm) : 151,11 (C6) ; 154,11 (C2) ; 149,91 (C4); 136,95 (C8) ; 113,46 (C5) ; 76,99 (C1') ; 72,58 (C4') ; 65,25 (C5') ; 56,95 (Ca) ; 51,81 (C2') ; 50,52 (C3') ; 30,04 (Cd) ; 23,76 (Cc) ; 20,36 (Cb).
- RNN ³¹P(U 400 Varian) : δ (ppm)) : -8,28 (d, 1P, γP) ; -8,97 (d, 1P, αP) ; -20,45 (t, 1P, βP).
- Spectrométrie de masse (appareil LCQ en mode négatif) : M - H⁻ = 576,9 g.mol⁻¹.
- Spectre UV : λmax = 252 nm.
- Electrophorèse capillaire :
   µep = -3,41 x 10⁻⁴ cm².V⁻¹.s⁻¹.

### Exemple 8 : Synthèse de la 4-(aminobutyl)-2-(cytosin-1-yl)-6-(hydroxyméthyl morpholine-6-triphosphate (morpholino C putrescine : 11

Toute la réaction est conduite à température ambiante, sous agitation magnétique, dans un ballon de 50 mL.

La réaction est la même que pour le composé **7**, à partir de la cytosine,5'-triphosphate (50 mg, 0,09 mmol, 1 éq.), de periodate de sodium (20 mg, 0,09 mmol, 1 éq.), de la putrescine (47 µL, 0,47 mmol, 5 éq), du borohydrure de sodium (au total 9,1 mg, 0,24 mmol, 2,5 éq.) ajouté en six fractions équivalentes, chacune dissoute dans 0,1 mL d'eau.

Une analyse par chromatographie en polarité de phase inversée (système O) : colonne Merck Lichrocart 125-4 LiChrospher 100RP-18 ("endcapped", 5 µm, 125x4 mm). Débit : 1 mL/min. Eluant : gradient TEAB 25 mM/MeOH, dans les conditions suivantes :

| t(min) | TEAB 25 mM(%) | MeOH (%) |
|---|---|---|
| 0 | 97 | 3 |
| 2 | 97 | 3 |
| 10 | 90 | 10 |
| 15 | 90 | 10 |
| 17 | 97 | 3 |

... **indique** un facteur de capacité k'= 4,18.

On purifie le composé **11** par précipitation au méthanol, puis passage sur 5 mL de résine Dowex sous forme Na⁺.

On obtient 47 mg de **11**, ce qui correspond à 85,4 % de rendement.
Caractérisation :
- RMN ¹H (AM 400 Brüker) : δ (ppm) : 7,78 (d, 1H, H6) ; 6,17 (d, 1H, H5) ; 5,96 (dd, 1H, H1') ; 4,22 (m, 1H, H4') ; 3,91 (m, 2H, H5', H5") ; 3,28 (m, 1H, H2"); 3,20 (m, 1H, H3") ; 3,16 (m, 2H, 2 Ha) ; 2,80 (m, 2H, 2 Hd) ; 2,44 (m, 1H, H2') ; 2,32 (m, 1H, H3') ; 1,79 (m, 4H, 2 Hb + 2 Hc)
- RMN ¹³C (AM 400 Brüker) : δ (ppm) : 166,056 (C4) ; 157,28 (C2) ; 142,43 (C6) ; 96,88 (C5) ; 80,57 (C1') ; 75,13 (C4') ; 66,48 (C5') ; 57,11 (Ca) ; 55,30 (C2') ; 52,45 (C3') 30,66 (Cd); 25,29 (Cc); 22,70 (Cb).
- RMN ³¹P (WM 250 Brüker) : δ (ppm) : -5,42 (d, 1P, γP) ; -10,06 (d, 1P, αP) ; -20,82 (m, 1P, βP).
- Spectrométrie de masse (appareil LCQ en mode négatif) : M - H⁻ = 536,0 g.mol⁻¹.
- Spectre UV : λmax = 268 nm
- Electrophorèse capillaire :
   µep = -2,99 x 10⁻⁴ cm².V⁻¹.s⁻¹.

### Exemple 9 : Synthèse de la 4-[5((2-aminobutyl)-thiouréidyl)fluorescein)]-2-(adénosin-9-yl)-6-(hydroxyméthyl)-morpholine-6-triphosphate (morpholino A putrescine-fluorescéine) 12.

Ce composé **12** répond à la formule (I) avec R¹ représentant l'adénine, et R² représentant (CH₂)₄NHR³ où R³ est un groupe dérivé de la fluorescéine.

Toutes les réactions sont conduites à la température ambiante, sous agitation magnétique, dans un ballon de 100 mL.

On ajoute en trois fois et progressivement à 200 mg (0,3 mmol, 1 éq.) de la Morpholino A putrescine **7** de l'exemple **5**, dans un mélange eau/pyridine (1/1), 184,9 mg (0,5 mmol, 1,5 éq.) de fluorescéine isothiocyanate. Le milieu est agité pendant 48 heures avant d'être évaporé à sec.

Une analyse par chromatographie en polarité de phase inversée sur la colonne Merck LiChrocart 125-4 LiChrospher 100 RP-18 ("endcapped", 5 µm, 125x4 mm), avec un débit de 1 mL/min en utilisant comme éluant : TEAA 25mM/MeOH [97/3], indique un rendement d'environ 48% (k'=7,51).
Purification : elle est faite par chromatographie "flash" sur une colonne de silice à polarité de phase inversée C-18 (Econosil prep 90, Alltech, France). L'éluant est un gradient eau/MeOH.
Caractérisation :
- RMN ¹H : δ (ppm) : 8,57 (s, 1H, H2), 8,31 (s, 1H, H8), 8,20-6,65 (9H, fluorescéine), 5,79 (dd, 1H, H1'), 4,25(m, 1H, H4'), 4,11 (m, 2H, H5', H5"), 3,60 (s, 2H, CH₂ putrescine), 3,12 (d, 1H, H3"), 2,93 (d, 1H, H2"), 2,81 (m, 1H, H2'), 2,59 (m, 2H, CH₂ putrescine), 2,50 (dd, 1H, H3'), 1,79 (s, 2H, CH₂ putrescine), 1,62 (m, 2H, CH₂ putrescine)
- RMN ³¹P : δ (ppm) : -8,45 (dd, 1P, γP), -13,25 (dd, 1P, αP), -24,20 (t, 1P, βP)
   Spectrométrie de masse : M-H⁻ = 949,2 g.mol⁻¹

### Exemple 10 : Synthèse de la 4-[5(((2-aminobutyl)-thioureidyl) fluorescein)1-2-(thymidin-1-yl)-6-(hydroxyméthyl) morpholine-6-triphosphate (morpholino T putrescine fluorescéine) 13.

Toutes les réactions sont conduites à température ambiante, sous agitation magnétique, dans un ballon de 25 mL.

A 30 mg (0,05 mmol, 1 éq) du composé 9 dissous dans 2 mL d'un mélange eau/pyridine (1/1), on ajoute en trois fois 31 mg (0,08 mmol, 1,5 éq.) de fluorescéine isothiocyanate. Le milieu est agité pendant 48 heures avant d'être évaporé à sec.

On purifie le composé **13** par chromatographie liquide hautes performances semi-préparative, sur une colonne à polarité de phases inversée (Système L) : colonne Macherey Nagel Nucléosil 7 C-18 (7 µm, 250 x 21 mm). Débit : 10 mL/min. Eluant : TEAB 25 mM/CH₃CN, dans les conditions suivantes :

| t(min) | TEAB 25 mM | CH₃CN |
|---|---|---|
| | (%) | (%) |
| 0 | 100 | 0 |
| 4 | 100 | 0 |
| 15 | 73 | 27 |
| 18 | 100 | 0 |

Caractérisation :
- Spectrométrie de masse (appareil LCQ en mode positif) : M-H⁺ = 942,1 g.mol⁻¹.
- Spectre UV : λmax = 488 nm.
- Electrophorèse capillaire :
   µcp = -4,23x10⁻⁴ cm².V⁻¹.s⁻¹.

### Exemple 11 : Synthèse de la 4-[5(((2-aminobutyl)-thioureidy fluorescein)]-2-(guanosin-9-yl)-6-(hydroxyméthyl) morpholine-6-triphosphate (morpholino G putrescine fluorescéine) 14.

Toutes les réactions sont conduites à température ambiante, sous agitation magnétique, dans un ballon de 25 mL.

A 30 mg (0,05 mmol, 1 éq.) du composé **10**, dissous dans 2 mL d'un mélange eau/pyridine (1/1), on ajoute en trois fois et progressivement 30 mg (0,08 mmol, 1,5 éq.) de fluorescéine isothiocyanate. Le milieu est agité pendant 48 heures avant d'être évaporé à sec.

Une analyse par chromatographie en polarité de phase inversée (système M) : colonne Merck-LiChrochart 125-4 LiChrospher 100 RP-18 (« endcapped », 5 µm, 125 x 4 mm). Débit : 1 mL/min. Eluant : gradient TEAB 25 mM/CH₃CN, dans les conditions suivantes :

| **t(min)** | **TEAB 25 mM** | **CH**_{**3**}**CN** |
|---|---|---|
| | (%) | (%) |
| 0 | 100 | 0 |
| 4 | 100 | 0 |
| 15 | 73 | 27 |
| 18 | 100 | 0 |

indique un rendement d'environ 24 % (k'= 4,62).

On purifie le composé **14** par chromatographie liquide hautes performances semi-préparative, sur une colonne à polarité inversée en utilisant le Système L de l'exemple 10.

On obtient 14,5 mg de composé **14**, soit un rendement de 30,0 %.
Caractérisation :
- RMN ¹H (AM 400 Brüker) : δ (ppm) : 7,87 (s, 1H, H8) ; 7,70-6,63 (9H, fluorescéine) ; 5,60 (dd, 1H, H1') ; 4,18 (m, 1H, H4') ; 4,12 (m, 2H, H5', H5") ; 3,82 (m, 1H, Ha) ; 3,61 (m, 1H, Ha) ; 3,08 (d, 1H, H3") ; 2,95 (d, 1H, H2") ; 2,82 (m, 1H, H2'); 2,71 (m, 1H, Hd) ; 2,55 (m, 1H, Hd) ; 2,39 (t, 1H, H3') ; 1,77 (m, 2H, 2 Hb) ; 1,62 (m, 2H, 2 Hc).
- RMN ¹³C (AM 400 Brüker) : δ (ppm) : 180,58 (plusieurs C fluorescéine) ; 158,37 (plusieurs C fluorescéine) ; 136,98 (C6) ; 131,06 (C2) ; 126,7 (C4) ; 122,85 (plusieurs C fluorescéine) ; 112,03 (C8) ; 103,80 (plusieurs C fluorescéine) ; 78,91 (C1'); 74,83 (C4') 65,96 (C5') ; 57,27 (Ca) ; 53,79 (C2') ; 52,56 (C3') ; 48,87 (Cd); 25,70 (Cc); 22,75 (Cb).
- RMN ³¹P (U 400 Varian) : δ (ppm) : -4,93 (dd, 1P, γP) ; -9,82 (d, 1P, αP) ; -19,94 (t, 1P, βP).
- Spectrométrie de masse (appareil LCQ en mode négatif): M - H ⁻ = 985,3 g.mol⁻¹.
- Spectre UV : λmax = 494 nm
- Electrophorèse capillaire :
   µep = -3,83 x 10⁻⁴ cm².V⁻¹ .s⁻¹.

### Exemple 12 : Synthèse de la 4-[5 (((2-aminobutyl)-thiouréidyl)fluorescein)]-2-(cytosin-1-yl)-6-(hydroxy méthyl)morpholino-6-triphosphate (morpholino C putrescine-fluorescéine) 15.

Toutes les réactions sont conduites à température ambiante, sous agitation magnétique, dans un ballon de 10 mL.

A 30 mg (0,05 mmol, 1 éq) du composé **11**, dissous dans 2 ml d'un mélange eau/pyridine (1/1), on ajoute en trois fois 36 mg (0,09 mmol, 1,5 éq.) de fluorescéine isothiocyanate. Le milieu est agité pendant 48 heures avant d'être évaporé à sec.

Une analyse par chromatographie en polarité de phase inversée (système M décrit dans l'exemple 11) indique un facteur de capacité k'=4,7.

On purifie le composé **15** par chromatographie liquide hautes performances semi-préparative, sur une colonne à polarité de phases inversée (Système L de l'exempl 10).

On obtient 22,7 mg du composé **15**, soit un rendement de 44,3%.
Caractérisation :
- RMN ¹H (AM 400 Brüker) : δ (ppm) : 7,99 (s, 1H, H6) ; 7,87-6,69 (9H, fluorescéine); 5,78 (d, 2H, H5 + H1') ; 4,14 (m, 1H, H4') ; 3,77 (m, 2H, H5', H5") ; 3,36 (m, 2H, 2 Ha) ; 3,32 (m, 1H, H2") ; 3,03 (m, 1H, H3") ; 2,81 (m, 1H, H2'); 2,69 (m, 2H, 2 Hd, 1,79) ; 2,30 (m, 1H, H3') ; 1,79 (m, 2H, 2 Hb) ; 1,68 (m, 2H, 2 Hc)
- RMN ¹³C (AM 400 Brüker) : δ (ppm) : 175,06 (plusieurs C fluorescéine) ; 157,62 (C2) ; 141,39 (plusieurs C fluorescéine) ; 131,56 (C6) ; 121,06 (plusieurs C fluorescéine) ; 114,60 (plusieurs C fluorescéine) ; 103,30 (plusieurs C fluorescéine) ; 96,53 (C5) ; 79,10 (C1'); 73,67 (C4') ; 65,42 (C5') ; 58,89 (Ca) ; 57,19 (C2') ; 51,78 (C3') ; 46,61 (Cd); 25,48 (Ce) ; 21,38 (Cb)
- RMN ³¹P(U 400 Varian ) : δ (ppm) : -2,97 (d, 1P, γP) ; -7,54 (d, 1P, αP) ; -18,56 (m, 1P, βP).
- Spectrométrie de masse (appareil LCQ en mode négatif) : M - H⁻ = 925,2 g.mol⁻¹.
- Spectre UV : λmax = 491 nm.
- Electrophorèse capillaire :
   µep = -4,26 x 10 cm².V⁻¹.s⁻¹.

### Exemple 13 : Utilisation du morpholino T glycine pour l'analyse d'une séquence d'ADN.

On teste le morpholino T glycine **4** de l'exemple 2 en réaction de séquence avec des amorces fluorescentes (Applied Biosystems, Perkin-Elmer, Foster City, CA, USA) sur une matrice standard qui est un ADN plasmidique Bluescript (Stratagene, La Jolla, CA, USA). L'enzyme utilisée est une Taq Polymérase (Perkin-Elmer) que l'on utilise dans son tampon (tampon TACS, Perkin-Elmer).

On effectue deux réactions avec le morpholino T glycine à 200 et 500 µM (tableau 4), ainsi que deux réactions témoins (tableau 5) avec le didésoxynucléotide T (Boehringer).

Le milieu réactionnel d'un volume total de 10 µL contient 125 ng de matrice, 1,25 pmoles d'amorce fluorescente et les autres constituants donnés dans les tableaux 4 et 5. Le milieu est soumis à des cycles thermiques afin de réaliser en nombre des molécules de brins d'ADN néoformés. Une amplification sur un appareil Perkin-Elmer 9700 est réalisée, selon les séquences suivantes : 3 min., 95°C ; 15 cycles (15 sec., 95°C ; 15 sec., 55°C ; 1 min., 70°C) ; 15 cycles (15 sec., 95°C ; 1 min., 70°C). Le produit d'amplification est purifié sur colonne de Sephadex G50.

La migration du produit d'amplification obtenu dans l'éluat de colonne est faite en gel dénaturant (urée 7M) d'acrylamide de type Long Ranger (6%), en TBE 1X, sur un appareil Applied Biosystems 377. L'électrophorèse se déroule pendant 12 heures sous 1500 V.

La préparation de la solution stock de nucléotides représentant ici un mélange des quatre nucléosides triphosphates naturels, appauvri en thymidine triphosphate (appelé dTTP mix) est effectuée de la façon suivante.

On mélange 2µL d'une solution 1,25 mM de dTTP (Promega) avec 2µL de dATP 5 mM (Promega), 2µL de dCTP 5 mM (Promega) et 2 µL de dGTP 5 mM (Promega).

**Tableau 4**

| | **Morpholino T glycine 200 µM** | **Morpholino T glycine 500 µM** |
|---|---|---|
| Tampon TACS (x5) | 2 µL | 2 µL |
| Z1M13 Primer (JOE) | 1 µL | 1 µL |
| DTTP mix | 1 µL | 1 µL |
| Morpholino T glycine 2 mM | 1 µL | 2,5 µL |
| Taq (3U/µL) | 1 µL | 1 µL |
| Matrice | 1 µL | 1 µL |
| H₂O | 3 µL | 1,5 µL |

**Tableau 5**

| | **ddTTP 250 µM** | **ddTTP 300 µM** |
|---|---|---|
| Tampon TACS (x5) | 2 µL | 2 µL |
| Z1M13 Primer (ROX) | 1 µL | 1 µL |
| DTTP mix | 1 µL | 1 µL |
| DdTTP 2,5 mM | 1 µL | 2,5 µL |
| Taq (3U/µL) | 1 µL | 1 µL |
| Matrice | 1 µL | 1 µL |
| H₂O | 3 µL | 1,5 µL |

On détecte les produits des réactions de séquence par fluorescence. Les résultats obtenus sont représentés sur la figure annexée qui illustre la détection des produits dans le gel de séquençage analysés par le logiciel Perkin Elmer Analysis, version 3.0.

Pour chaque essai, les primers sont identifiables par leurs propriétés de fluorescence, le marqueur ROX (rouge) pour la réaction de contrôle avec le didésoxythymidine triphosphate 250 µm (courbe en tirets) et le marqueur JOE (vert) pour la réaction concernant la morpholino T glycine 200 µm (courbe en trait plein).

Comme le montre la figure, les résultats de ces tests sont tout à fait concluants puisque le morpholino T glycine est bien incorporé de manière base spécifique par la Taq Polymérase, et agit bien comme un terminateur de chaîne.

Les trois autres morpholino-nucléotides **1**, **5** et **6** peuvent être employés de la même manière pour déterminer les positions des quatre bases de l'ADN dans le fragment à analyser.

### Exemple 14 : Test des morpholino A putrescine et morpholino A fluorescéine en séquençage.

On teste les morpholino A putrescine (MATPP) **7** et morpholino A fluorescéine (MATPPF) **12** en réaction de séquence avec des amorces fluorescentes (Applied Biosystems, Perkin-Elmer, Foster City, CA, USA) sur une matrice standard qui est un ADN plasmidique Bluescript (Stratagene, La Jolla, CA, USA). L'enzyme utilisée est une Taq Polymérase (Perkin-Elmer) que l'on utilise dans son tampon (tampon Thermo Sequenase, Amersham, Life Science).

On effectue trois réactions de séquence avec le MATPP à 100, 200 et 400 µM et quatre réactions de séquence avec le MATPPF à 200, 500, 1000 et 5000 µM, ainsi que des réactions témoins avec le didésoxynucléotide ddATP à la concentration de 250 µM (Boehringer).

Le milieu réactionnel d'un volume total de 10 µL contient 125 ng de matrice, 1,25 pmoles d'amorce fluorescente et les autres constituants tel que décrit dans les tableaux.

Le milieu est soumis à des cycles thermiques afin de réaliser en nombre des molécules de brins d'ADN néoformés. Une amplification sur un appareil Perkin-Elmer 9700 (Gene Amp®, PCR System 9700) est réalisée, selon les séquences suivantes :
**MATPP 7** 3 min, 95°C ; 30 cycles (15 sec., 95°C ; 15 sec., 55°C ; 1 min, 70°C)
**MATPPF 12** 3 min, 95°C ; 30 cycles (15 sec., 95°C ; 15 sec., 55°C ; 4 min, 60°C)

Les produits d'amplification sont purifiés sur colonne de Sephadex G50. Les produits de chaque réaction de séquence sont mélangés aux produits d'une réaction témoin et analysés par électrophorèse.

La migration du mélange obtenu est faite en gel dénaturant (urée 7M) d'acrylamide de type Long Ranger (6%), en TBE 1X, sur un appareil Applied Biosystems 377 (ABI Prism DNA Sequencer, Perkin Elmer). L'électrophorèse se déroule pendant 7 heures sous 1680 V, 50 mA.

### Préparation de la solution stock de nucléotides : dATP mix pour 16 réactions

représentant ici un mélange des quatre nucléotides triphosphates naturels, appauvri en désoxyadénosine triphosphate (appelé dATP mix) : on mélange 4µL d'une solution 1,25 mM de dATP (Promega) avec 4µL de dTTP 5 mM (Promega), 4µL de dCTP 5 mM (Promega) 4 µL de dGTP 5 mM (Promega).

**Tableau 6**

| **Préparation du Mix commun pour 15 réactions** | | |
|---|---|---|
| | /réaction | /15 réactions |
| Tampon TACS (x5) | 2 µL | 30 µL |
| dATP mix | 1 µL | 15 µL |
| Taq (5U/µL) | 1 µL | 15 µL |
| Matrice (plasmide Bluescript) | 2 µL | 30 µL |

### Préparation de la solution mère 2mM de MATPP 7 :

(1,17 mg de MATPP **7** est dilué dans 1,04 ml d'H₂O.

**Tableau 7**

| **Réactions avec la morpholino ATPP 2mM** | | | |
|---|---|---|---|
| | **Morpholino ATPP 400 µM** | **Morpholino ATTP 200 µM** | **Morpholino ATTP 100 µM** |
| Mix commun | 6 µL | 6 µL | 6 µL |
| Z1M13 Primer (JOE) | 1 µL | 1 µL | 1 µL |
| Morpholino ATPP 2 mM | 2 µL | 1 µL | 0,5 µL |
| H₂O | 1 µL | 2 µL | 2,5 µL |

**Tableau 8**

| **Trois réactions de contrôle avec le didésoxyadénosine triphosphate (ddATP) 2,5 mM** | |
|---|---|
| | **DdATP 250 µM** |
| Mix commun | 6 µL |
| Z1M13 Primer (ROX) | 1 µL |
| DdATP 2,5 mM | 1 µL |
| H₂O | 2 µL |

### Préparation des solutions mères 20 mM et 2mM de MATPPF 12

Solution S₀ à 20 mM : diluer l'échantillon (2,2 mg) dans 110,5 µL d'H₂O

Solution S₁ à 2 mM : préléver 10 µL de S₀ et rajouter 90 µL d'H₂O

**Tableau 9**

| **Réactions avec la morpholino ATPPF 20 mM (S**_{**0**}**)** | | |
|---|---|---|
| | **MATPPF 1000 µM** | **MATTPF 5000 µM** |
| Mix commun | 6 µL | 6 µL |
| Z1M13 Primer (JOE) | 1 µL | 1 µL |
| Morpholino ATPPF 20 mM | 0,5 µL | 2,5 µL |
| H₂O | 2,5 µL | 0,5 µL |

**Tableau 10**

| **Réactions avec la morpholino ATPPF 2 mM (Si)** | | |
|---|---|---|
| | **MATPPF 500 µM** | **MATTPF 200 µM** |
| Mix commun | 6 µL | 6 µL |
| Z1M13 Primer (JOE) | 1 µL | 1 µL |
| Morpholino ATPPF 2 mM | 2,5 µL | 1 µL |
| H₂O | 0,5 µL | 2 µL |

**Tableau 11**

| **Quatre réactions de contrôle avec le didésoxyadénosine triphosphate (ddATP) 2,5 mM** | |
|---|---|
| | **ddATP 250 µM** |
| Mix commun | 6 µL |
| Z1M13 Primer (ROX) | 1 µL |
| ddATP 2,5 mM | 1 µL |
| H₂O | 2 µL |

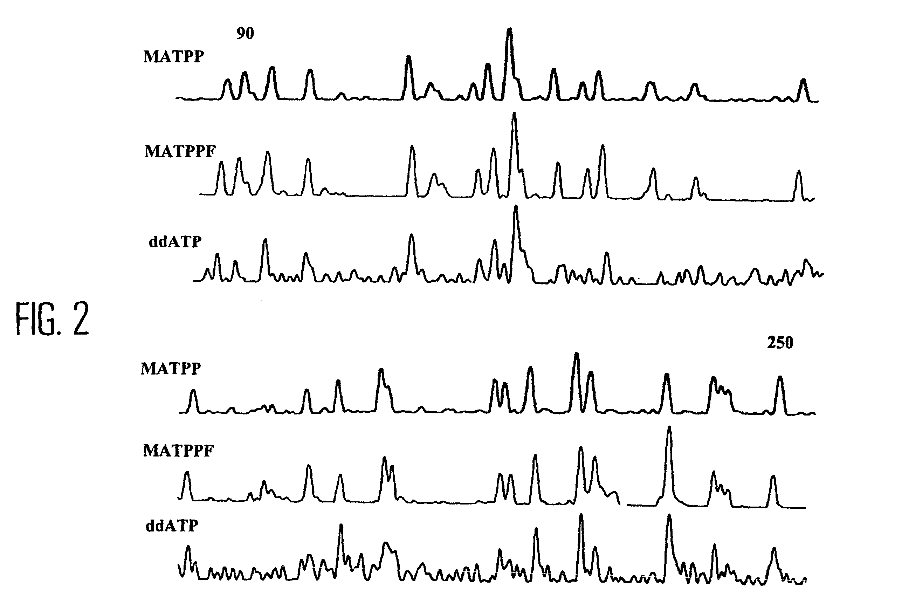

On donne sur la figure 2 les résultats obtenus avec le morpholino A putrescine **7** à 100 µM et le morpholino A fluorescéine **12** à 5 mM, entre la 90^{ème} et la 250^{ème} base.

On constate donc que ces deux dérivés agissent bien comme des terminateurs de chaîne. De plus, il est à noter que les réactions effectuées avec le dérivé fluorescent, le morpholino A fluorescéine, ont été détectées par le fluorophore porté par ce dérivé : on a donc préparé un terminateur de chaîne fluorescent.

### Exemple 15 : Utilisation des morpholino A putrescine (MATPP) et morpholino A fluorescéine (MATPPF) pour le marquage matrice-dépendant de fragments d'ADN en 3' ; test de l'incorporation enzymatique de ces composés par trois polymérases (Taq, Klenow, Klenow Exo Free) et une transcriptase inverse.

Ces deux dérivés de nucléosides triphosphates sont testés en incorporation enzymatique pour marquer un oligonucléotide de 13 bases de long en son extrémité 3'. Ce marquage est dit "matrice dépendant" car les enzymes utilisées ont besoin de la cible complémentaire pour allonger l'oligonucléotid selon les règles de Watson & Crick. La séquence A (17870 pmol/mL) étudiée ainsi que sa cible C (16128 pmol/mL) sont données dans la figure ci-dessous :

Cible C :                    3'-TGC CAA CCA ACC CCA CCT CAA CCT CTG-5'
Amorce A :                 5'-ACG GTT GGT TGG G (13 bp)
Fragments attendus :5'-ACG GTT TGG GGT GGA  (18 bp)
et longueurs (bp)      :5'-ACG GTT GGT TGG GGT GGA GTT GGA (24 bp)
                                5'-ACG GTT GGT TGG GGT GGA GTT GGA GA  (26 bp)
                                5'-ACG GTT GGT TGG GGT GGA GTT GGA  GAC (27 bp)

Trois enzymes sont utilisées pour ce marquage : la Taq DNA polymérase (Boehringer Mannheim), la Klenow (Boehringer Mannheim) et la Klenow Exonuclease Free (Amersham Life Science). L'amorce est marquée en son extrémité 5' par incorporation de ³²P phosphate avec le kit "Ready to go" T4 Polynucléotide Kinase (Pharmacia Biotech). L'amorce radiomarquée est notée A*.

On prépare les tampons de réaction des trois enzymes pour 10 réactions :

**Tableau 12**

| (en µL) | Réaction Taq | Réaction Klenow Exo Free |
|---|---|---|
| C | 50 | 50 |
| A | 10 | 10 |
| A* | 10 | 10 |
| Tp 10X | 50 | 50 |
| H₂O | 50 | 50 |

**Tableau 13**

| (en µL) | Réaction Klenow |
|---|---|
| C | 50 |
| A | 10 |
| A* | 10 |
| Tp 5X | 100 |
| H₂O | 0 |

Les enzymes sont ensuite diluées de la façon suivante, pour 10 réactions :
- Taq (5U/µL) : 10X0,1 µL de Taq + 10X15,5 µL d'H₂O
- Klenow (20U/µL) : 10X0,1 µL de Klenow + 10X15,5 µL d'H₂O
- Klenow Exo Free (5U/µL) : 10X0,1 µL de Klenow Exo Free + 10X15,5 µL d'H₂O.

On prépare également des solutions contenant les nucléosides triphosphates normaux :
- Solution "2P" composée d'un mélange de dGTP et dTTP à 0,1 mM chacun
- Solution "4P" composée d'un mélange de dATP, dCTP, dGTP et dTTP à 0,1 mM chacun

Les réactions de mises en oeuvres sont décrites dans le tableau 14 suivant :

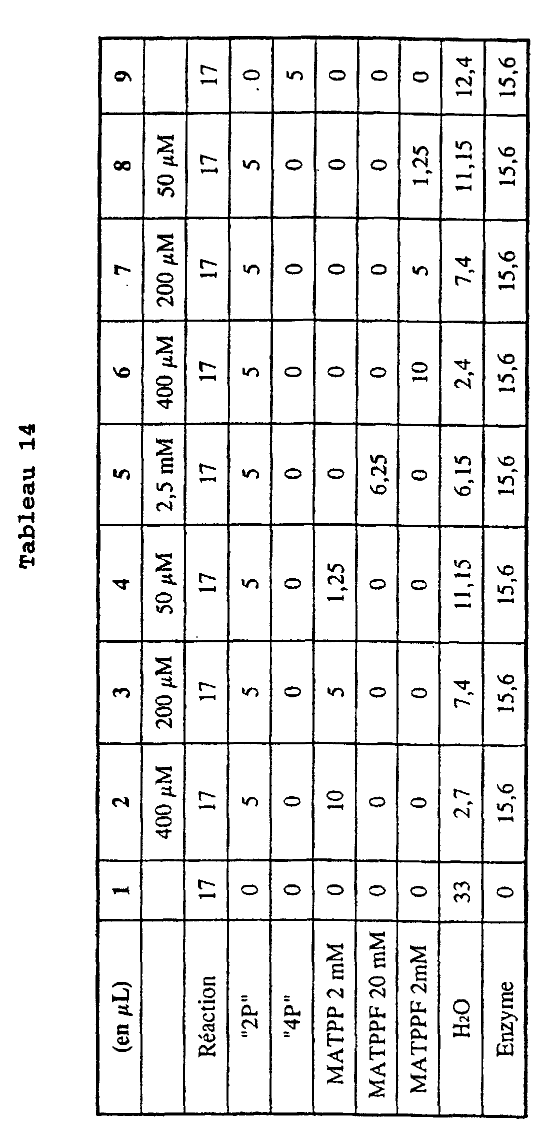

Le morpholino A putrescine est ainsi testé à trois concentrations : 400, 200 et 50 µM tandis que le morpholino A fluorescéine est mis en réaction à 2,5 mM, 400, 200 et 50 µM.

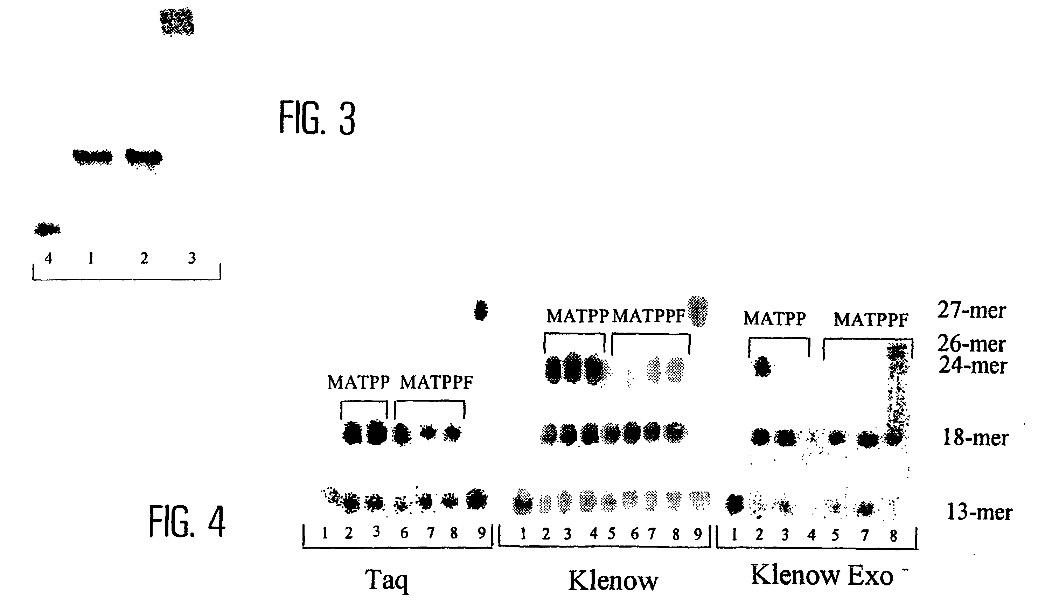

Avant l'ajout de l'enzyme, le mélange est dénaturé à 94°C pendant 5 minutes. Puis on laisse revenir à température ambiante afin que l'hybridation ait lieu. L'élongation est effectuée à 70°C pour la Taq, 37°C pour les deux Klenow, et ce pendant 10 minutes. Enfin, le milieu est à nouveau dénaturé par une solution de formamide et chauffage à 90 °C pendant 5 minutes avant d'être déposé sur un gel de polyacrylamide. La séparation est effectuée par électrophorèse à 2000 V. La lecture du gel est faite au Phosphorimager ; les résultats obtenus sont montrés sur la figure 3.

Sur cette figure, les pistes numéro 1 servent de contrôle de migration de l'oligonucléotide A marqué. Cet oligonucléotide a une longueur de 13 bases (13-mer). Les pistes 2, 3 et 4 permettent de suivre l'allongement de l'oligonucléotide A et l'incorporation du morpholino A putrescine. Dans ces conditions, seuls les nucléotides dGTP et dTTP (solution "2P") ont été ajoutés et sont utilisables par l'enzyme pour procéder à l'extension de l'amorce. La présence du morpholino A putrescine dans le milieu réactionnel permet son incorporation au niveau de la base 18. Un témoin a été effectué, en ne mettant dans le milieu que le mélange "2P" ; dans ce cas, l'enzyme poursuit son extension jusqu'à la 17^{ème} base puisqu'elle n'a pas de dérivé de l'adénosine pour continuer sa polymérisation. Ainsi, la différence de migration entre ce témoin, long de 17 bases, et les réactions 2, 3 et 4 confirme l'incorporation du MATPP et l'interruption de l'élongation de la chaîne. Les réactions 5 à 8 correspondent aux mêmes réactions avec le morpholino A fluorescéine. Là encore, le MATPPF est bien incorporé et arrête la polymérisation du brin complémentaire. On note toutefois pour les deux Klenow, qu'il y a eu parfois incorporation d'une autre base (G ou T) à la place du dérivé morpholino. En effet, on trouve dans ces cas des produits d'élongation, correspondant aux 18- et 24-mer.

Le puits 9 (voir figure 4) est une réaction de contrôle : le milieu réactionnel contient les 4 désoxynucléotides normaux et peut de ce fait allonger l'amorce jusqu'à son extension maximale, c'est-à-dire jusqu'à obtention du 27-mer.

En conclusion, les trois enzymes incorporent le morpholino A putrescine et le morpholino A fluorescéine dans toutes les concentrations testées, y compris aux plus faibles concentrations.

La capacité des transcriptases inverses à incorporer les dérivés morpholinonucléotides au cours de l'extension d'oligonucléotides à été confirmée. Dans ce test, la transcriptase inverse (M-MLV, Promega ; activité : 200 000 U/mL) est choisie comme modèle. Cette dernière est capable de synthétiser un brin d'ADN complémentaire d'une cible (ADN ou ARN), à partir d'une amorce oligonucléotide, en présence de nucléosides triphosphates. On teste donc les morpholino A putrescine et morpholino A fluorescéine à des concentrations finales de 250 µM. Une copie de contrôle est aussi déposée sur le gel, avec les quatre nucléosides triphosphates de la solution "4P".

La séquence de la cible C (27-mer, 16128 pmol/mL) est celle de l'amorce B (14-mer, 56368 pmol/mL) sont montrées ci-dessous. Cette amorce B, marquée de façon radioactive est notée B*.

La solution B* contient alors 10 pmol de l'amorce B dans un volume de 50 µL. On dilue également les solutions de C et B dix fois ; ces solutions sont notées respectivement C/10 et B/10.

Cible C : 3'-TGC CAA CCA ACC CCA CCT CAA CCT CTG-5'
Amorce B : 5'-ACG GTT GGT TGG GG (14 bp)

**Tableau 15**

| (en µL) | Réaction 1 | Réaction 2 | Réaction 3 | Réaction 4 |
|---|---|---|---|---|
| C/10 | 2 | 2 | 2 | 0 |
| B* | 5 | 5 | 5 | 5 |
| B/10 | 3 | 3 | 3 | 0 |
| Tampon 5X | 4 | 4 | 4 | 0 |
| MATPP 2 mM | 2,5 | 0 | 0 | 0 |
| MATPPF 2 mM | 0 | 2,5 | 0 | 0 |
| "2P" | 2,5 | 2,5 | 0 | 0 |
| "4P" | 0 | 0 | 2,5 | 0 |
| H₂O | 0 | 0 | 0 | 15 |
| Enzyme | 1 | 1 | 1 | 0 |

Comme précédemment, le mélange est dénaturé, avant l'ajout de l'enzyme, à 94°C pendant 5 minutes et on laisse revenir à température ambiante. L'élongation se fait à 37°C pendant 60 minutes. Le milieu est dénaturé par une solution de formamide et chauffage à 90 °C pendant 5 minutes avant d'être déposé sur un gel de polyacrylamide. La séparation se fait par électrophorèse à 1500 V. La lecture du gel est faite au Phosphorimager ; les résultats obtenus sont donnés sur la figure 4.

Sur cette figure, la piste 4 permet d'estimer la longueur de l'amorce B marquée. La piste 3 montre l'allongement maximal de l'amorce B jusqu'à un produit final de 27 paires de bases en présence des 4 désoxynucléotides naturels. Les réactions 1 et 2 montrent que les dérivés morpholino sont incorporés au cours de l'élongation de l'amorce B par la transcriptase inverse. Cette incorporation est quantitative et fournit un produit de 18 paires de bases (en absence de dérivé morpholino, l'extension est bloquée à la 17^{ème} base).

En conclusion, les dérivés morpholino sont très bien reconnus par la transcriptase inverse et incorporés dans les amorces en cours d'allongement selon un processus base spécifique.

### Références citées

- [1] :: Sanger et al, Proceedings of National Academy of Science, 74, 1977, p. 5463-5467.
- [2] :: WO-A-96/23807.
- [3] :: Prober et al, Science, 238, 1987, pages 336-341.
- [4] :: Hileman et al, Bioconjugate Chemistry, 5, 1994, pages 436-444.
- [5] :: Broker et al, Nucleic Acids Research, 5, 1978, pages 363-385.
- [6] :: Agrawal et al, Nucleic Acids Research, 14, 1986, pages 6227-6245.
- [7] :: FR-A-2 710 068
- [8] :: Rayford et al, Journal of Biological Chemistry, 260, 1985, pages 15708-15713.

## Revendications

1. Procédé de fabrication d'un fragment d'acide nucléique (ADN ou ARN) marqué en 3', qui comprend l'incorporation enzymatique d'un dérivé de nucléotide ayant pour précurseur un composé de formule : 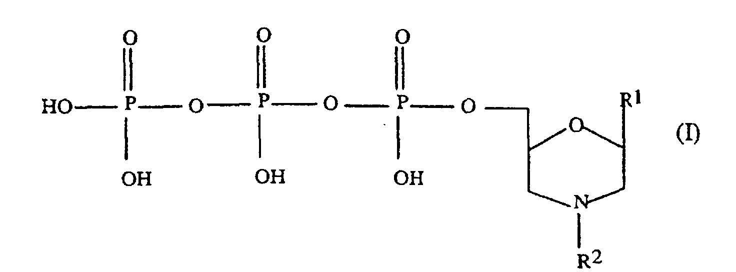 dans laquelle R¹ représente une base nucléique et R² représente un groupe répondant à l'une des formules suivantes :
-(CH₂)ₙ-NH₂ -(CH₂)ₙ-SH
-(CH₂)ₙ-COOH -(CH₂)ₙ-OH
- (CH₂)ₙ-NH-R³ -(CH₂)ₙ-SR³
- (CH₂)ₙ-CO-R³ -(CH₂)ₙ-OR³
dans lesquelles n est un nombre entier allant de 1 à 12 et R³ est un groupe dérivé d'un marqueur, d'une protéine, d'une enzyme, d'un acide gras ou d'un peptide, à l'extrémité 3' OH du fragment d'acide nucléique.

2. Procédé de modification d'un fragment d'acide nucléique par incorporation enzymatique en 3' d'un morpholino-nucléotide modifié ayant pour précurseur un composé répondant à la formule : 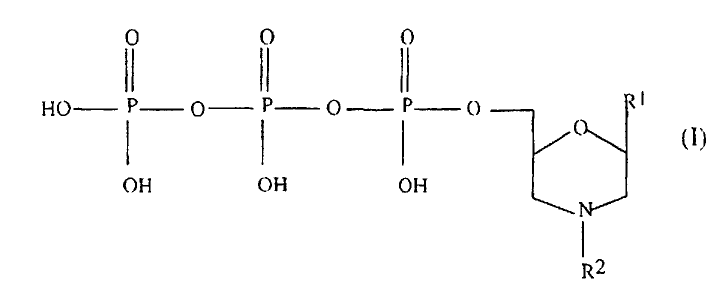 dans laquelle R¹ représente une base nucléique et R² représente un groupe répondant à l'une des formules suivantes :
-(CH₂)ₙ-NH-R³
-(CH₂)ₙ-CO-R³
- (CH₂)ₙ-SR³
- (CH₂)ₙ-OR³
dans lesquelles n est un nombre entier allant de 1 à 12 et R³ représente un composé choisi parmi les photoréticulants, les acides gras, les peptides hydrophobes, les anticorps, les enzymes et les fluorophores.

3. Procédé de séquençage d'un acide nucléique (ADN ou ARN) par la technique de polymérisation enzymatique de la séquence complémentaire de cet acide nucléique en utilisant des terminateurs de chaînes, dans lequel au moins l'un des terminateurs de chaînes a pour précurseur un composé répondant à la formule : 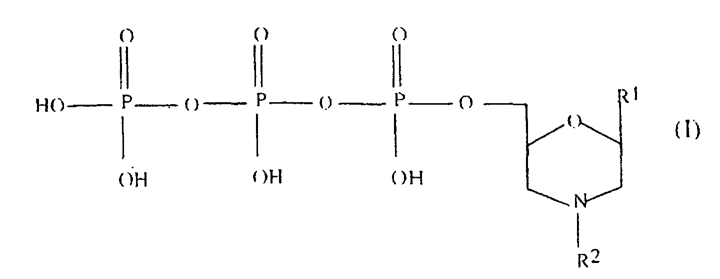 dans laquelle R¹ représente une base nucléique et R² représente un groupe répondant à l'une des formules suivantes :
-(CH₂)ₙ-NH₂ -(CH₂)ₙ-SH
- (CH₂)ₙ-COOH -(CH₂)ₙ-OH
-(CH₂)ₙ-NH-R³ -(CH₂)ₙ-SR³
- (CH₂)ₙ-CO-R³ -(CH₂)ₙ-OR³
dans lesquelles n est un nombre entier allant de 1 à 12 et R³ est un groupe dérivé d'un marqueur, d'une protéine, d'une enzyme, d'un acide gras ou d'un peptide.

4. Procédé selon la revendication 1, 2 ou 3, dans lequel l'enzyme est le fragment de Klenow de l'ADN polymérase.

5. Procédé selon la revendication 1, 2 ou 3, dans lequel l'enzyme est une polymérase thermorésistante d'une bactérie Thermophile ou la terminale transférase ou la transcriptase inverse.

6. Procédé selon l'une quelconque des revendications 1 à 5, dans lequel la base nucléique est une base nucléique naturelle choisie parmi l'adénine, la guanine, la cytosine, la thymine, l'uracile, la xanthine, l'hypoxanthine, la 2-aminopurine et leurs dérivés.

7. Procédé selon l'une quelconque des revendications 1 à 5, dans lequel R¹ répond à l'une de formules suivantes :

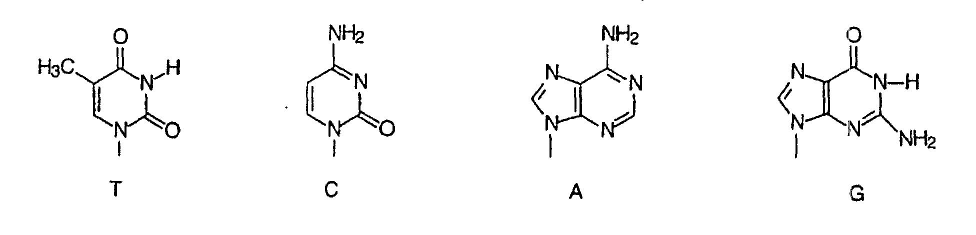

8. Procédé selon l'une quelconque des revendications 1 à 7, dans lequel le marqueur est choisi parmi les produits radioactifs, les produits luminescents, électroluminescents et fluorescents, les molécules capables de se coupler à d'autres molécules, les molécules autorisant des interactions du type antigène-anticorps, et les marqueurs enzymatiques.

9. Procédé selon l'une quelconque des revendications 1 à 7, dans lequel R³ est un fluorophore.

10. Procédé selon la revendication 9, dans lequel R³ est choisi parmi les dérivés de la fluorescéine, de la biotine et de la rhodamine.

11. Procédé selon la revendication 1, 2 ou 3 dans lequel le dérivé, le morpholino-nucléotide modifié, ou le terminateur de chaîne est le composé (I) sous forme de monophosphate.

12. Morpholino-nucléotide répondant à la formule : 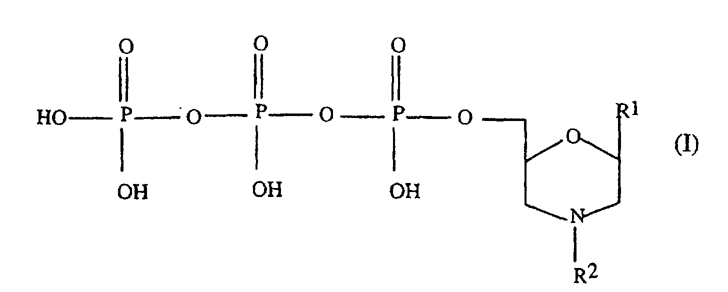 dans laquelle R¹ est l'adénine et R² représente -CH₂-COOH, -(CH₂)₄-NH₂ ou -(CH₂)₄-NH-R³ avec R³ représentant un groupe dérivé de la fluorescéine.

13. Morpholino-nucléotide de formule : 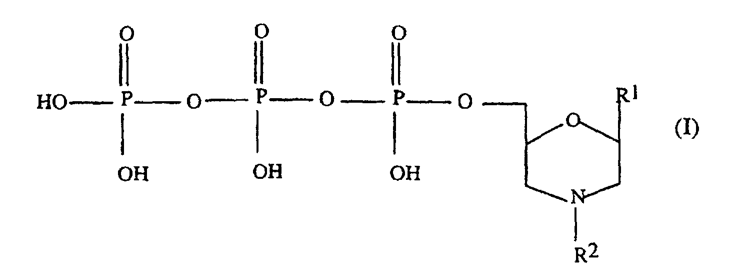 dans laquelle R¹ est la thymine et R² représente -CH₂-COOH, -(CH₂)₄-NH₂ ou -(CH₂)₄-NH-R³ avec R³ représentant un groupe dérivé de la fluorescéine.

14. Morpholino-nucléotide répondant à la formule : 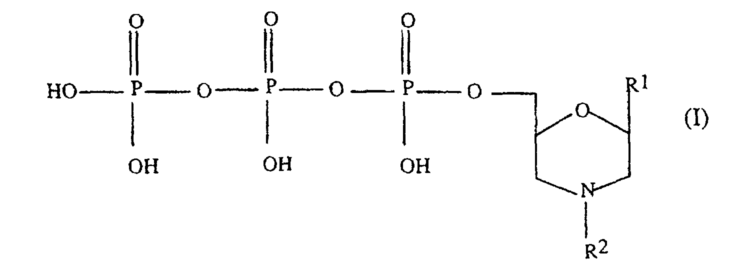 dans laquelle R¹ est la cytosine et R² représente -CH₂-COOH, -(CH₂)₄-NH₂ ou -(CH₂)₄-NH-R³ avec R³ représentant un groupe dérivé de la fluorescéine.

15. Morpholino-nucléotide répondant à la formule : 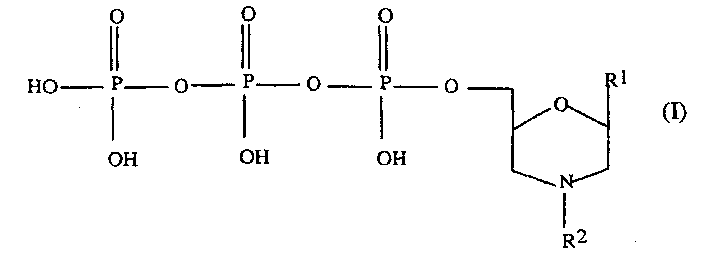 dans laquelle R¹ est la guanine et R² représente -CH₂-COOH, -(CH₂)₄-NH₂ ou -(CH₂)₄-NH-R³ avec R³ représentant un groupe dérivé de la fluorescéine.

16. Procédé de fabrication d'un morpholino-nucléotide de formule : 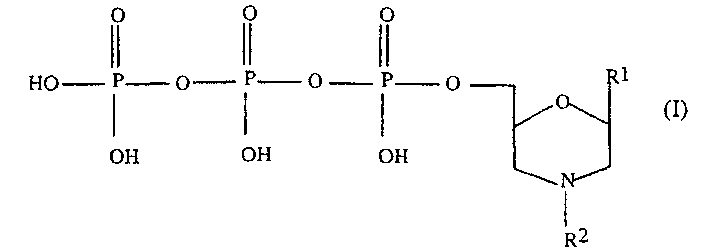 dans laquelle R¹ représente une base nucléique et R² représente un groupe répondant à l'une des formules suivantes :
-(CH₂)ₙ-NH₂ -(CH₂)ₙ-SH
-(CH₂)ₙ-COOH -(CH₂)ₙ-OH
- (CH₂)ₙ-NH-R³ -(CH₂)ₙ-SR³
- (CH₂)ₙ-CO-R³ -(CH₂)ₙ-OR³
dans lesquelles n est un nombre entier allant de 1 à 12 et R³ est un groupe dérivé d'un marqueur, d'une protéine, d'une enzyme, d'un acide gras ou d'un peptide, la réaction d'un nucléoside triphosphate de formule : 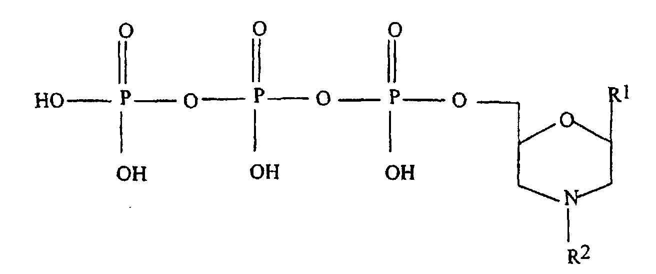 dans laquelle R¹ a la signification donnée ci-dessus, avec un periodate, un composé de formule R² NH₂ dans laquelle R² a la signification donnée ci-dessus et du borohydrure de sodium.

17. Utilisation d'un morpholino-nucléotide de formule : 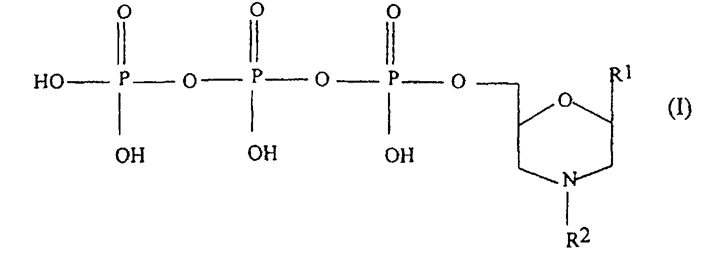 dans laquelle R¹ représente une base nucléique et R² représente un groupe répondant à l'une des formules suivantes :
-(CH₂)ₙ-NH₂ -(CH₂)ₙ-SH
-(CH₂)ₙ-COOH -(CH₂)ₙ-OH
-(CH₂)ₙ-NH-R³ -(CH₂)ₙ-SR³
- (CH₂)ₙ-CO-R³ -(CH₂)ₙ-OR³
dans lesquelles n est un nombre entier allant de 1 à 12 et R³ est un groupe dérivé d'un marqueur, d'une protéine, d'une enzyme, d'un acide gras ou d'un peptide, pour le marquage de fragments d'ADN ou d'ARN.

## Claims

1. Process for manufacturing a 3'-labelled nucleic acid (DNA or RNA) fragment, which comprises the enzymatic incorporation of a nucleotide derivative having as precursor a compound of formula: 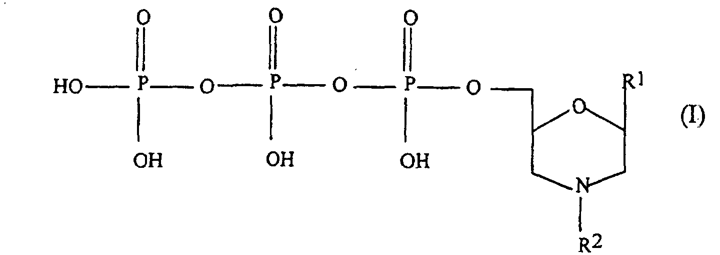 in which R¹ represents a nucleic base and R² represents a group corresponding to one of the following formulae:.
-(CH₂)ₙ-NH₂ -(CH₂)ₙ-SH
-(CH₂)ₙ-COOH -(CH₂)ₙ-OH
- (CH₂)ₙ-NH-R³ -(CH₂)ₙ-SR³
- (CH₂)ₙ-CO-R³ -(CH₂)ₙ-OR³
in which n is an integer ranging from 1 to 12 and R³ is a group derived from a label, a protein, an enzyme, a fatty acid or a peptide, at the 3' OH end of the nucleic acid fragment.

2. Process for modifying a nucleic acid. fragment by enzymatic incorporation at the 3' end of a modified morpholino nucleotide having as precursor a compound corresponding to the formula: 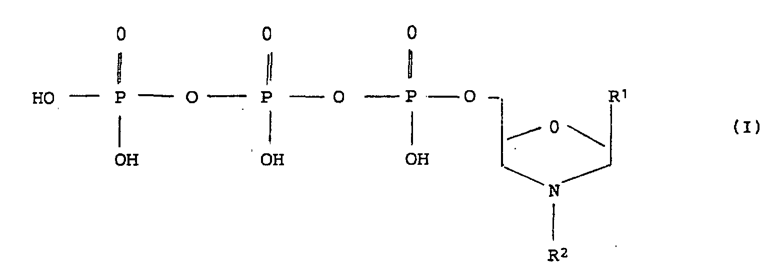 in which R¹ represents a nucleic base and R² represents a group corresponding to one of the following formulae:
- (CH₂)ₙ-NH-R³
- (CH₂)ₙ-CO-R³
- (CH₂)ₙ-SR³
- (CH₂)ₙ-OR³
in which n is an integer from 1 to 12 and R³ represents a compound chosen from photo-crosslinking agents, fatty acids, hydrophobic peptides, antibodies, enzymes and fluorophores.

3. Process for sequencing a nucleic acid (DAN or RNA) by the technique of enzymatic polymerization of the sequence complementary to this nucleic acid using chain terminators, in which at least one of the chain terminators has as precursor a compound corresponding to the formula: 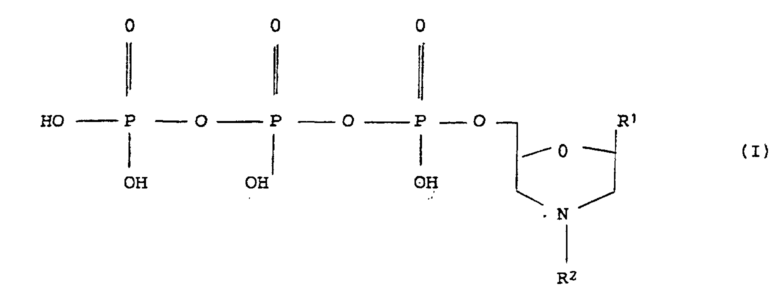 in which R¹ represents a nucleic base and R² represents a group corresponding to one of the following formulae:
-(CH₂)ₙ-NH₂ -(CH₂)ₙ-SH
-(CH₂)ₙ-COOH -(CH₂)ₙ-OH
- (CH₂)ₙ-NH-R³ -(CH₂)ₙ-SR³
-(CH₂)ₙ-CO-R³ -(CH₂)ₙ-OR³
in which n is an integer ranging from 1 to 12 and R³ is a group derived from a label, a protein, an enzyme, a fatty acid or a peptide.

4. Process according to Claim 1, 2 or 3, in which the enzyme is the Klenow fragment of DNA polymerase.

5. Process according to Claim 1, 2 or 3, in which the enzyme is a heat-resistant polymerase of a Thermophilus bacterium or terminal transferase or reverse transcriptase.

6. Process according to one of Claims 1 to 5, in which the nucleic base is a natural nucleic base chosen from adenine, guanine, cytosine, thymine; uracil, xanthine, hypoxanthine and 2-aminopurine, and derivatives thereof.

7. Process according to any one of Claims 1 to 5, in which R¹ corresponds to one of the following formulae:

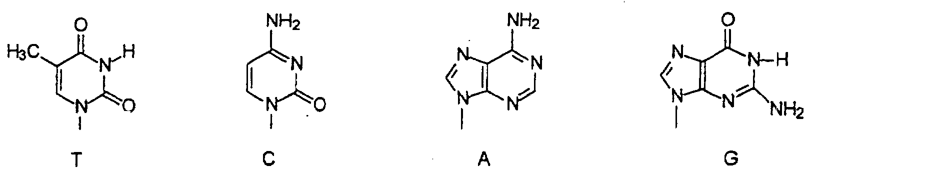

8. Process according to one of Claims 1 to 7, in which the label is chosen from radioactive products, luminescent products, electroluminescent and fluorescent products, molecules capable of coupling with other molecules, molecules which allow interactions of the antigen-antibody type, and enzymatic labels.

9. Process according to any one of the claims 1 to 7, in which R³ is a fluorophore.

10. Process according to Claim 9, in which R³ is chosen from fluorescein derivatives, biotin derivatives and rhodamine derivatives.

11. Process according to Claim 1, 2 or 3, in which the derivative, the modified morpholino-nucleotide or the chain terminator is compound (I) in monophosphate form.

12. Morpholino-nucleotide corresponding to the formula: 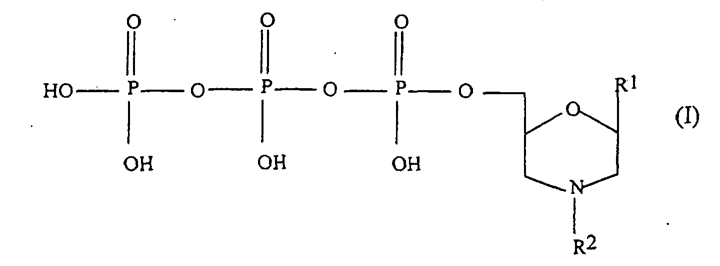 in which R¹ is adenine and R² represents -CH₂-COOH, -(CH₂)₄-NH₂ or -(CH₂)₄-NH-R³ with R³ representing a group derived from fluorescein.

13. Morpholino-nucleotide of formula: 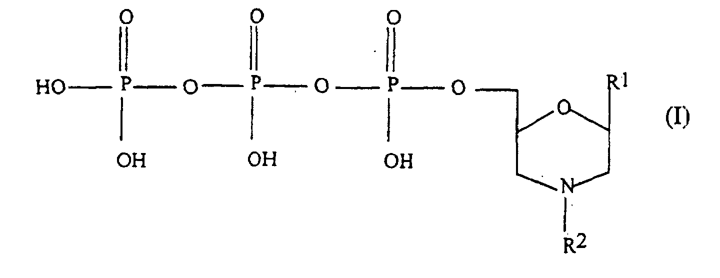 in which R₁ is thymine and R² represents -CH₂-COOH, -(CH₂)₄-NH₂ or -(CH₂)₄-NH-R³ with R³ representing a group derived from fluorescein.

14. Morpholino-nucleotide corresponding to the formula: 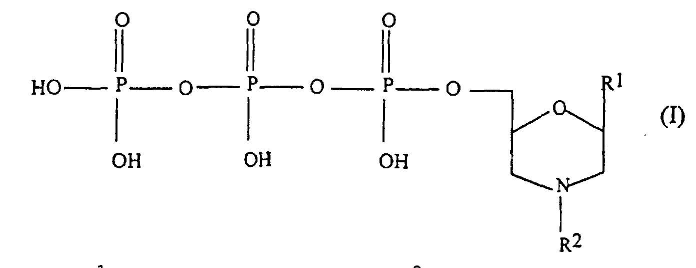 in which R¹ is cytosine and R² represents -CH₂-COOH, -(CH₂)₄-NH₂ or -(CH₂)₄-NH-R³ with R³ representing a group derived from fluorescein.

15. Morpholino-nucleotide corresponding to the formula: 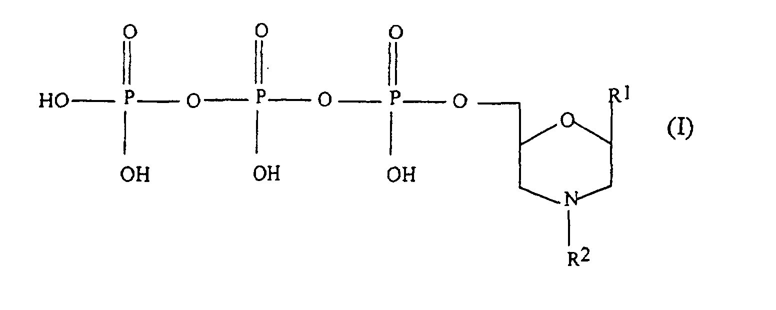 in which R¹ is guanine and R² represents -CH₂-COOH, -(CH₂)₄-NH₂ or -(CH₂)₄-NH-R³ with R³ representing a group derived from fluorescein.

16. Process for manufacturing a morpholino-nucleotide of formula: 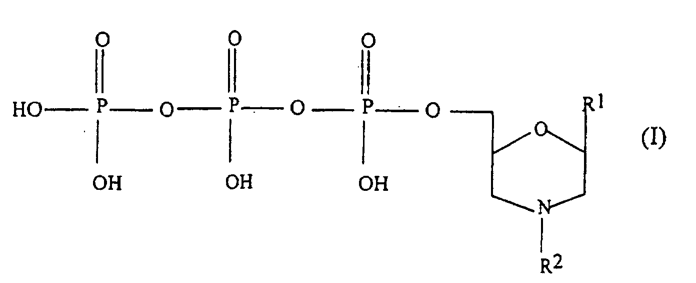 in which R¹ represents a nucleic base and R² represents a group corresponding to one of the following formulae:
-(CH₂)ₙ-NH₂ -(CH₂)ₙ-SH
- (CH₂)ₙ-COOH -(CH₂)ₙ-OH
- (CH₂)ₙ-NH-R³ -(CH₂)ₙ-SR³
-(CH₂)ₙ-CO-R³ -(CH₂)ₙ-OR³
in which n is an integer ranging from 1 to 12 and R³ is a group derived from a label, from a protein, from an enzyme, from a fatty acid or from a peptide, the reaction of a nucleoside triphosphate of formula: 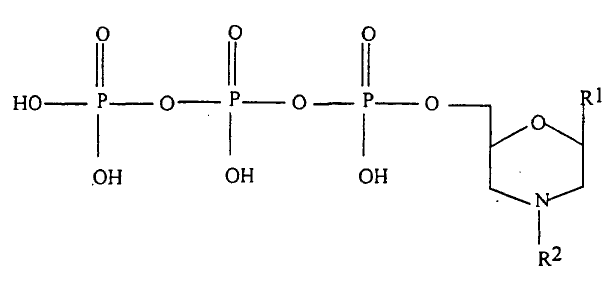 in which R¹ has the meaning given above, with a periodate, a compound of formula R² NH₂ in which R² has the meaning given above, and sodium borohydride.

17. Use of a morpholino-nucleotide of formula: 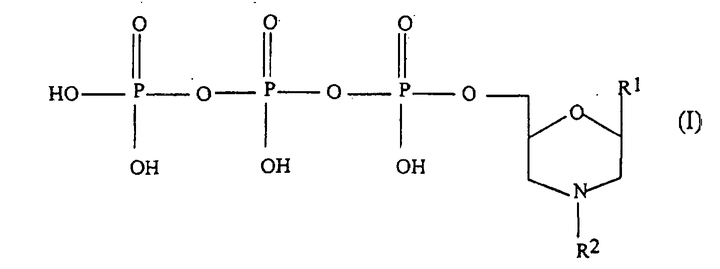 in which R¹ represents a nucleic base and R² represents a group corresponding to one of the following formulae:
-(CH₂)ₙ-NH₂ -(CH₂)ₙ-SH
-(CH₂)ₙ-COOH -(CH₂)ₙ-OH
-(CH₂)ₙ-NH-R³ -(CH₂)ₙ-SR³
- (CH₂)ₙ-CO-R³ -(CH₂)ₙ-OR³
in which n is an integer ranging from 1 to 12 and R³ is a group derived from a label, from a protein, from an enzyme, from a fatty acid or from a peptide, for the labelling of DNA or RNA fragments.

## Patentansprüche

1. Verfahren zur Herstellung eines in der 3'-Position markierten Nucleinsäure (DNA oder RNA-Fragments, das umfasst die enzymatische Einführung eines Nucleotid-Derivats, dessen Vorläufer eine Verbindung der Formel ist, in das 3'-OH-Ende des Nucleinsäure-Fragments 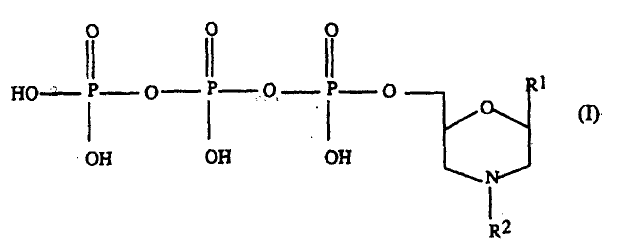 in der R¹ eine Nucleinbase und R² eine Gruppe einer der folgenden Formeln darstellen:
-(CH₂)ₙNH₂ -(CH₂)ₙSH
-(CH₂)ₙ-COOH -(CH₂)ₙ-OH
-(CH₂)ₙ-NH-R³ -(CH₂)ₙ-SR³
- (CH₂)ₙ-CO-R³ -(CH₂)ₙ-OR³
worin n steht für eine ganze Zahl von 1 bis 12 und R³ steht für eine Gruppe, die abgeleitet ist von einem Marker, einem Protein, einem Enzym, einer Fettsäure oder einem Peptid.

2. Verfahren zur Modifizierung eines Nucleinsäure-Fragments durch ehzymatische Einführung eines modifizierten Morpholinonucleotids, dessen Vorläufer eine Verbindung der Formel ist, in die 3'-Position: 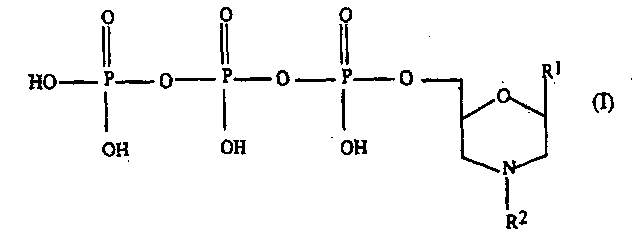 in der R¹ eine Nucleinbase und R² eine Gruppe einer der folgenden Formeln darstellen:
-(CH₂)ₙNH-R³
-(CH₂)ₙCO-R³
-(CH₂)ₙSR³
-(CH₂)ₙ-OR³
worin n steht für eine ganze Zahl von 1 bis 12 und R³ steht für eine Verbindung, ausgewählt aus der Gruppe der Lichtvemetzungsmittel, der Fettsäuren, der hydrophoben Peptide, der Antikörper, der Enzyme und der Fluorophoren.

3. Verfahren zum Sequenzieren einer Nudeinsäuren (DNA oder RNA) nach der Methode der enzymatischen Polymerisation der komplementären Sequenz dieser Nucleinsäure unter Verwendung von Ketten-Terminatoren, bei dem eine Verbindung der nachstehend angegebenen Formel einen Vorläufer für mindestens einen der Ketten-Terminatoren darstellt: 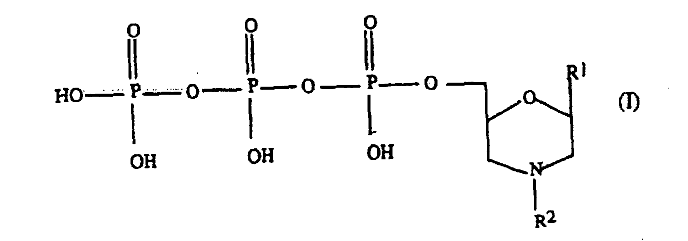 in der R¹ eine Nucleinbase und R² eine Gruppe einer der folgenden Formeln darstellen:
-(CH₂)ₙNH₂ -(CH₂)ₙSH
-(CH₂)ₙ-COOH -(CH₂)ₙ-OH
-(CH₂)ₙ-NH-R³ -(CH₂)ₙ-SR³
-(CH₂)ₙ-CO-R³ -(CH₂)ₙ-OR³
worin n steht für eine ganze Zahl von 1 bis 12 und R³ steht für eine Gruppe, die abgeleitet ist von einem Marker, einem Protein, einem Enzym, einer Fettsäure oder einem Peptid.

4. Verfahren nach Anspruch 1, 2 oder 3, in dem das Enzym das; Klenow-Fragment der DNA-Polymerase ist.

5. Verfahren nach Anspruch 1, 2 oder 3, in dem das Enzym eine wärmebeständige Polymerase eines thermophilen Bakteriums oder die terminals Transferase oder die inverse Transkriptase ist.

6. Verfahren nach einem der Ansprüche 1 bis 5, in dem die Nucleinbase eine natürliche Nucleinbase ist, ausgewählt aus der Gruppe Adenin, Guanin, Cytosin, Thymin, Uracil, Xanthin, Hypoxanthin, 2-Aminopurin und ihren Derivaten.

7. Verfahren nach einem der Ansprüche 1 bis 5, in dem R¹ einer der folgenden Formeln entspricht:

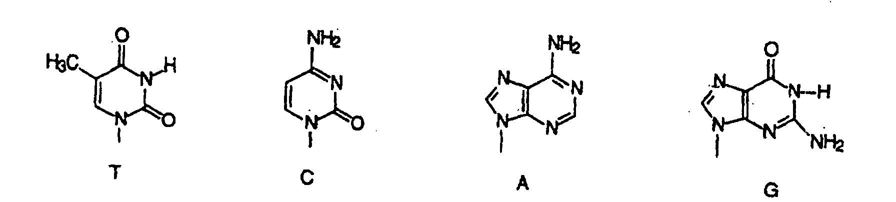

8. Verfahren nach einem der Ansprüche 1 bis 7, in dem der Marker ausgewählt wird aus der Gruppe der radioaktiven Produkte, der lumineszierenden, elektrolumineszierenden und fluoreszierenden Produkte, der Moleküle, die sich an andere Moleküle ankoppeln können, der Moleküle, die Wechselwirkungen vom Antigen-Antikörper-Typ ermöglichen, und der enzymatischen Marker.

9. Verfahren nach einem der Ansprüche 1 bis 7, in dem R³ für einen Fluorophoren steht.

10. Verfahren nach Anspruch 9, in dem R³ ausgewählt wird unter den Derivaten von Fluorescein, Biotin und Rhodamin.

11. Verfahren nach Anspruch 1, 2 oder 3, in dem das Derivat, das modifizierte Morpholino-Nucleotid oder der Ketten-Terminator die Verbindung (I) in Form des Monophosphats ist.

12. Morpholino-Nucleotid der Formel: 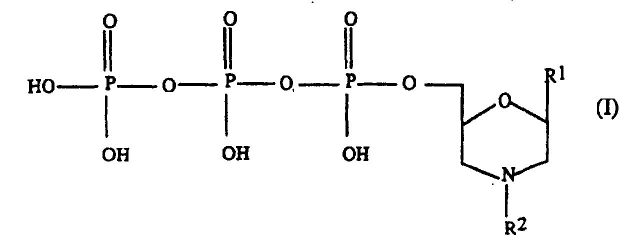 in der R¹ Adenin und R² -CH₂-COOH, -(CH₂)₄-NH₂ oder -(CH₂)₄-NH-R³ darstellen, worin R³ für eine von Fluorescein abgeleitete Gruppe steht.

13. Morpholino-Nucletoid der Formel: 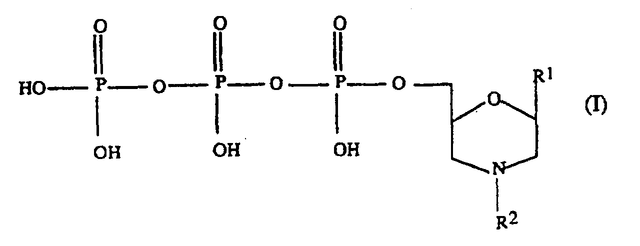 in der R¹ Thymin und R² -CH₂-COOH, -(CH₂)₄-NH₂ oder -(CH₂)₄-NH-R³ darstellen, worin R³ für eine von Fluorescein abgeleitete Gruppe steht.

14. Morpholino-Nucletoid der Formel: 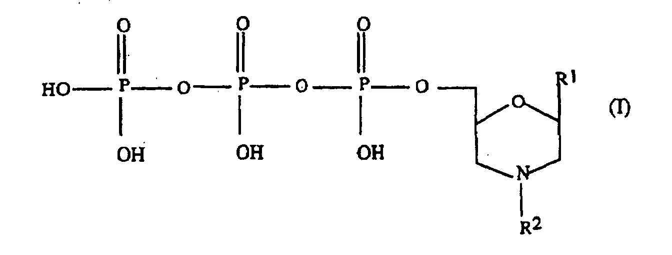 in der R¹ Cytosin und R² -CH₂-COOH, -(CH₂)₄-NH₂ oder -(CH₂)₄-NH-R³ darstellen, worin R³ für eine von Fluorescein abgeleitete Gruppe steht.

15. Morpholino-Nucletoid der Formel: 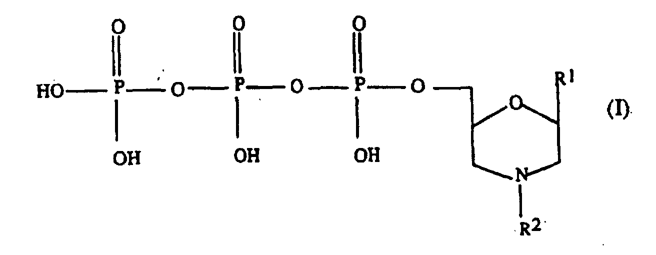 in der R¹ Guanin und R² -CH₂-COOH, -(CH₂)₄-NH₂ oder -(CH₂)₄-NH-R³ bedeuten, worin R³ für eine von Fluorescein abgeleitete Gruppe steht.

16. Verfahren zur Herstellung eines Morpholino-Nucletoids der Formel: 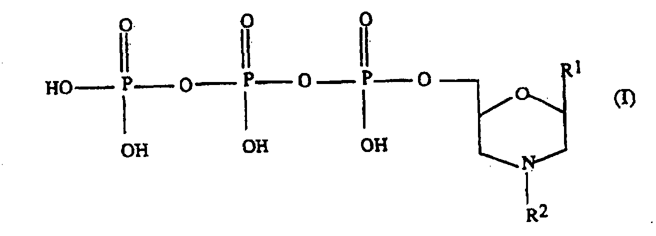 in der R¹ eine Nucleinbase und R² eine Gruppe einer der folgenden Formeln darstellen:
-(CH₂)ₙNH₂ -(CH₂)ₙSH
-(CH₂)ₙ-COOH -(CH₂)ₙ-OH
-(CH₂)ₙ-NH-R³ -(CH₂)ₙ-SR³
-(CH₂)ₙ-CO-R³ -(CH₂)ₙ-OR³
worin n steht für eine ganze Zahl von 1 bis 12 und R³ steht für eine Gruppe, die von einem Marker, einem Protein, einem Enzym, einer Fettsäure oder einem Peptid abgeleitet ist,
durch Umsetzung eines Nucleosidtriphosphats der Formel 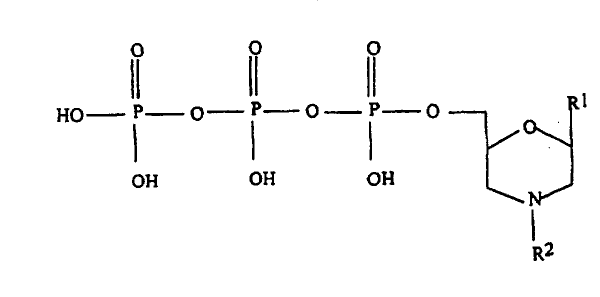 in der R¹ die oben angegebene Bedeutung hat,
mit einem Periodat, einer Verbindung der Formel R²-NH₂, in der R² die oben angegebene Bedeutung hat, und Natriumborhydrid.

17. Verwendung eines Morpholinonucleotids der Formel: 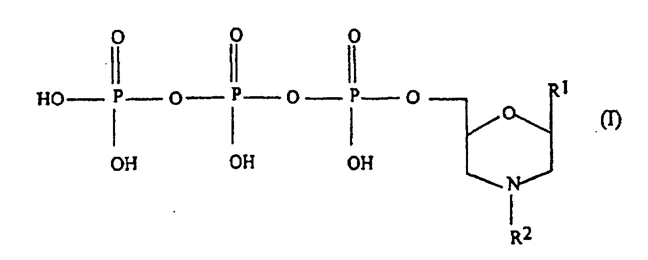 in der R¹ eine Nucleinbase und R² eine Gruppe einer der folgenden Formeln darstellen:
-(CH₂)ₙNH₂ -(CH₂)ₙSH
-(CH₂)ₙ-COOH -(CH₂)ₙ-OH
-(CH₂)ₙ-NH-R³ -(CH₂)ₙ-SR³
-(CH₂)ₙ-CO-R³ -(CH₂)ₙ-OR³
worin n steht für eine ganze Zahl von 1 bis 12 und R³ steht für eine Gruppe, die von einem Marker, einem Protein, einem Enzym, einer Fettsäure oder einem Peptid abgeleitet ist,
zur Markierung von DNA- oder RNA-Fragmenten.
